# FASCICULE DE BREVET EUROPEEN

(11) **EP 3 265 809 B1**
(45) Date de publication et mention de la délivrance du brevet: **29.05.2019**
(21) Numéro de dépôt: 16709316.0
(22) Date de dépôt: 04.03.2016
(51) Int. Cl.: G01N 33/50, G01N 33/543, G01N 33/53

(54) **PROCÉDÉ ET DISPOSITIF POUR DÉTECTER EN TEMPS RÉEL UN COMPOSÉ SÉCRÉTÉ ET LA CIBLE SÉCRÉTRICE AINSI QUE LEURS UTILISATIONS**
VERFAHREN UND VORRICHTUNG ZUR ECHTZEITERFASSUNG EINER SEKRETIERTEN VERBINDUNG UND DES SEKRETIONSZIELS SOWIE VERWENDUNGEN DAVON
METHOD AND DEVICE FOR DETECTING IN REAL TIME A SECRETED COMPOUND AND THE SECRETION TARGET, AND USES THEREOF

(30) Priorité: 06.03.2015 FR 1551918
(43) Date de publication de la demande: 10.01.2018
(73) Titulaire: Commissariat à l'Énergie Atomique et aux Énergies Alternatives, 75015 Paris (FR); Centre National de la Recherche Scientifique, 75016 Paris (FR)
(72) Inventeur: ROUPIOZ, Yoann, 38570 Goncelin (FR); LEROY, Loïc, 38000 Grenoble (FR); MARCHE, Patrice, 38240 Meylan (FR); DELLEA, Olivier, 42350 La Talaudière (FR); CUBIZOLLES, Myriam, 38700 Corenc (FR); HERRIER, Cyril, 22120 Yffiniac (FR); LIVACHE, Thierry, 38560 Jarrie (FR)
(74) Mandataire: Brevalex
(86) Numéro de dépôt international: PCT/EP2016/054685
(87) Numéro de publication internationale: WO 2016/142301

(56) Documents cités:
- EP-A1- 2 600 149
- WO-A1-02/097122
- WO-A1-2008/106048
- WO-A1-2012/073202
- WO-A2-2014/074540
- FR-A1- 2 985 520
- US-A1- 2007 134 743
- US-B1- 6 410 252
- MILGRAM S ET AL: "On chip real time monitoring of B-cells hybridoma secretion of immunoglobulin", BIOSENSORS AND BIOELECTRONICS, ELSEVIER BV, NL, vol. 26, no. 5, 15 janvier 2011 (2011-01-15), pages 2728-2732, XP027580230, ISSN: 0956-5663 [extrait le 2010-09-29]
- SHIRASAKI Y ET AL: "Single cell real time secretion assay using amorphous fluoropolymer microwell array", 2011 16TH INTERNATIONAL SOLID-STATE SENSORS, ACTUATORS AND MICROSYSTEMS CONFERENCE (TRANSDUCERS 2011) : BEIJING, CHINA, 5 - 9 JUNE 2011, IEEE, PISCATAWAY, NJ, 5 juin 2011 (2011-06-05), pages 755-758, XP031910671, DOI: 10.1109/TRANSDUCERS.2011.5969330 ISBN: 978-1-4577-0157-3

## Description

### DOMAINE TECHNIQUE

La présente invention concerne le domaine des procédés et dispositifs de détection appliquée à la biologie et notamment le domaine de la détection multiple appliquée à la biologie.

En effet, la présente invention concerne un procédé et des dispositifs particuliers permettant non seulement de déterminer et de quantifier les sécrétions cellulaires mais aussi d'identifier le ou les type(s) de cellules concernées, les utilisations de tels procédé et dispositifs ainsi que les procédés de préparation de ces dispositifs.

### ÉTAT DE LA TECHNIQUE ANTÉRIEURE

La communication entre cellules est un événement biologique de première importance. Les modes de communication peuvent avoir différentes formes comme un contact physique entre cellules ou bien l'émission de composés chimiques par une cellule et la réception de ces composés par une autre cellule.

Ce dernier mode correspond à un mode de communication chimique dans laquelle l'information du message est portée par un ensemble de paramètres tels que la nature, la quantité, la fréquence et la durée de sécrétion des composés, le type cellulaire de la cellule émettrice, le type cellulaire de la cellule réceptrice (ou cellule « cible »).

Ainsi, les activités sécrétrices des cellules font l'objet de nombreuses investigations biomédicales pour, par exemple, suivre et comprendre l'ensemble des cascades immunitaires impliquées dans le développement des infections, des cancers, des maladies auto-immunes, des allergies, de l'impact des vaccins ou dans le traitement des rejets de greffe. La possibilité de suivre le déclenchement d'une réponse immunitaire est donc un enjeu important pour de nombreuses applications biomédicales diagnostiques et thérapeutiques.

L'ensemble des techniques décrites à ce jour faisant appel à un protocole séquentiel qui comprend une incubation, puis une révélation et une lecture en point final, il n'existe pas de test rapide permettant de détecter des sécrétions cellulaires en quelques minutes, voire même en quelques heures. D'autre part, les étapes de marquage (fluorescent et/ou révélation colorimétrique) limitent également le nombre de paramètres (recherche de différentes cytokines par exemple) qui peuvent être suivis à partir d'un même échantillon (problème de recouvrement des spectres dans le visible et/ou d'excitation/émission dans l'UV ou de déconvolution de signaux composés de différentes couleurs). Enfin, un corollaire de ces étapes de marquage indispensable est l'impossibilité de suivre, en temps réel, les cinétiques de sécrétion dans le milieu extracellulaire.

Un point important à souligner dans la mise en oeuvre des techniques actuellement disponibles est la nécessité absolue de passer par des étapes de marquage et une lecture *a posteriori,* i.e. en point final. Ce mode de production de résultat ne donne pas d'information en temps réel sur les sécrétions et ne permet donc pas d'accéder aux paramètres cinétiques des interactions tels que constantes d'association, et dissociation, profils cinétiques de réponses en fonction des cytokines et/ou des types cellulaires....

A l'heure actuelle, plusieurs dispositifs d'analyse permettent de déterminer/quantifier les sécrétions cellulaires issues d'un ou plusieurs types de cellules.

Parmi ces dispositifs d'analyse, on peut citer le test de diagnostic T-SPOT®.TB (Oxford Immunotec) notamment décrit dans la demande internationale WO 98/23960 **[1]** qui permet l'analyse de la réponse immunitaire à l'échelle cellulaire pour diagnostiquer la tuberculose. Ce test se base sur la technique dite ELISPOT **[2]** qui implique la culture, dans des plaques multipuits, d'échantillons cellulaires pendant des intervalles de temps de quelques heures à quelques jours, suivie du lavage de ces puits (élimination des cellules sécrétrices), et d'une succession d'étapes de lavage/révélation (test sandwich ELISA). Le fond de ces puits étant recouvert d'anticorps spécifiques d'un composé sécrété, un montage moléculaire à l'aide d'un deuxième anticorps suivi d'une révélation colorimétrique permet de visualiser *a posteriori* des tâches ou « spots » à l'emplacement où une cellule a sédimenté pendant la culture et a sécrété un composé dans le milieu de culture. La diffusion des composés dans l'environnement proche d'une cellule permet donc de faire apparaître des taches caractéristiques d'une activité sécrétrice cellulaire individuelle.

Shirasaki et al, 2011 (Transducers 2011, pages 755-758) propose un test permettant d'observer, en temps réel, la sécrétion d'interleukine 1β par des cellules uniques vivantes grâce à l'utilisation de micropuits fonctionnalisés par des anticorps anti-IL-1β et d'anticorps de détection marqués par des points quantiques fluorescents.

D'autres dispositifs mettent en oeuvre des techniques utilisant la cytométrie en flux.

Ainsi, dans la technique ICS pour « IntraCellular Staining », les membranes cellulaires sont perméabilisées pour marquer les cytokines à l'intérieur de la cellule à l'aide d'anticorps couplés à des fluorophores **[3].** Ce type de manipulations suivi d'un passage en cytométrie en flux permet une analyse individuelle des cellules et peut permettre de trier/sélectionner des cellules. D'une part, la perméabilisation des membranes affecte significativement la viabilité des cellules, et empêche toute remise en culture ultérieure des cellules sélectionnées. De plus, dans ce système, la mesure détecte la présence de la cytokine à l'intérieur de la cellule sans pouvoir affirmer que cette dernière est réellement secrétée. En d'autres termes, cette technique ne fournit qu'une donnée indicative puisque la présence d'une protéine dans le milieu intracellulaire ne prévaut pas systématiquement sa présence dans le milieu extracellulaire.

La technique d'encapsulation de cellules dans des gels d'agarose fonctionnalisés par des anticorps spécifiques des molécules sécrétées est également basée sur une analyse en cytométrie en flux par détection de fluorescence émise par des montages moléculaires en sandwich **[4].** Des billes gélifiées contenant une cellule sécrétrice sont alors fluorescentes et peuvent être triées par cytométrie en flux.

Le test de Miltenyi Biotec repose sur la combinaison de 4 anticorps différents permettant de croiser les informations caractéristiques du type cellulaire sécréteur et de la cytokine libérée dans le milieu extracellulaire. Avant leur analyse individuelle en cytométrie en flux, les cellules sont marquées à leur surface par un anticorps spécifique d'un marqueur membranaire permettant l'identification de leur type cellulaire. Cet anticorps est lui-même conjugué de façon covalente à un second anticorps, formant un anticorps chimère, spécifique de la cytokine d'intérêt. Si celle-ci est sécrétée dans le milieu extracellulaire, elle est alors captée par ce second anticorps sur lequel un troisième anticorps (spécifique d'un autre épitope de la même cytokine) peut se complexer et être révélé par la reconnaissance d'un quatrième anticorps anti-espèce spécifique des immunoglobulines produites chez l'espèce hôte du troisième anticorps [5]. Cette approche souffre toutefois d'une mise en oeuvre complexe et coûteuse en particulier dans le cas de la recherche simultanée de différentes cytokines.

La technique de « *patch-clamp* » qui permet de quantifier, par mesure de capacitance et/ou ampérométrique, des composés électriquement chargés, sécrétés par la partie de la membrane plasmique obstruant la pipette n'est pas applicable pour l'étude simultanée d'un grand nombre de cellules **[6].** Il s'agit surtout d'étudier les processus physico-chimiques en jeu au cours de la sécrétion à l'échelle d'une cellule et notamment à l'échelle macromoléculaire de la membrane.

On peut citer encore d'autres méthodes permettant de déterminer et éventuellement quantifier les sécrétions cellulaires issues d'un ou plusieurs types cellulaires telles que :
- des méthodes électrochimiques **[7]** qui, bien que très sensibles, peuvent être complexes et coûteuses à mettre en oeuvre ;
- des méthodes impliquant un compartimentage microfluidique comme décrite dans la demande internationale WO 2011/056643 **[8]** où des cellules individuelles sont introduites par manipulation microfluidique dans des microchambres de réaction ;
- des méthodes impliquant une plateforme opto-fluidique en transmission **[9]** avec une analyse plasmonique simultanément couplée à une analyse électrochimique ; ces méthodes ne permettent toutefois pas de différencier les cellules et la validité biologique des résultats peut être faussée du fait que l'environnement *in vivo* du matériel biologique est peu ou pas simulé ; et
- des méthodes impliquant une plateforme de phénotypage; la demande internationale WO 2006/060646 **[10]** propose une caractérisation génique de sécrétions et une analyse de mécanisme de régulation génique interne à la ou aux cellule(s) ; ces méthodes ne permettent toutefois pas une analyse en temps réel et la validité biologique des résultats peut être faussée du fait que l'environnement *in vivo* du matériel biologique est peu ou pas simulé.

Enfin, le dispositif décrit dans **[11]** met en oeuvre l'imagerie de résonance de surface (SPRi) et un support d'analyse constitué d'un prisme de verre recouvert sur une face par une couche d'or sur laquelle se trouve(nt) un ou plusieurs type(s) de ligands correspondant à une surface de détection. Le principe consiste à éclairer, au travers du prisme et sous différents angles (variation de l'angle thêta), la surface de détection avec un faisceau polarisé et à analyser les variations du faisceau réfléchi causées par l'interaction de l'onde évanescente créée par résonance plasmon et le milieu analysé. La longueur de pénétration de l'onde évanescente étant de l'ordre de quelques centaines de nm, la méthode n'est alors sensible qu'aux variations d'indice de réfraction du milieu, très proches de la surface d'or (i.e. à l'adsorption spécifique ou non de molécules à la surface fonctionnelle de l'or). L'analyse simultanée d'une surface de 1 cm² par imagerie permet l'observation de motifs spécifiques à différents composants. Toutefois, dans cette configuration et dans celle décrite dans la demande de brevet US 2013/137085 [12], le signal des cellules se superpose au signal des sécrétions, ce qui peut nuire à la qualité d'analyse du fait de l'absence de discrimination entre les deux signaux.

Milgram et al, 2011 (Biosensors and Bioelectronics, vol. 26, pages 2728-2732) utilisent également la résonance plasmonique de surface pour détecter, en temps réel, la sécrétion d'immunoglobulines par des hybridomes de lymphocytes B.

De plus, jusqu'à aujourd'hui, l'analyse du sécrétome i.e. le milieu contenant les sécrétions mais pas les cellules est souvent déportée de la zone de reconnaissance des cellules, entraînant l'impossibilité d'associer le type cellulaire et la sécrétion **[13].**

Pour résumer, les outils, i.e. les procédés et les dispositifs, disponibles à ce jour sont quasiment tous dédiés à des mesures mono-paramétriques ou multiparamétriques avec un seul type de sécrétion recherchée en point final et ce, avec un très bas débit.

Les présents inventeurs se sont donc fixé pour but de proposer un procédé et un dispositif, industrialisables, permettant de déterminer et de détecter, de façon simple, rapide et en temps réel, à la fois le composé sécrété et la cellule sécrétrice et ce, sans perte de sensibilité.

### EXPOSÉ DE L'INVENTION

La présente invention permet de résoudre des problèmes techniques et inconvénients précédemment listés. En effet, les inventeurs proposent un procédé et des dispositifs permettant le suivi, en temps réel, de sécrétions cellulaires par un groupe de cellules ou des cellules individuelles. Un tel procédé donne l'accès à deux nouvelles propriétés de la sécrétion cellulaire - en plus de la caractérisation qualitative et quantitative - qui sont, d'une part, le suivi des cinétiques de réponse et, d'autre part, de la variation intercellulaire au sein d'un échantillon complexe.

En particulier, la présente invention propose un procédé pour mesurer en temps réel la sécrétion d'un composé Cₒ par une cible Cᵢ, ledit procédé comprenant :
- la mise en culture en milieu liquide, dans une chambre de culture, d'une pluralité de cibles parmi lesquelles se trouve au moins une cible Cᵢ, ladite chambre de culture présentant :
   i) au moins une 1^{ère} surface sur laquelle ladite cible Cᵢ est présente, la présence de ladite cible Cᵢ sur ladite 1^{ère} surface générant un 1^{er} signal, et
   ii) au moins une 2^{nde} surface, différente de ladite 1^{ère} surface et non coplanaire avec ladite 1^{ère} surface, fonctionnalisée par au moins un ligand se liant spécifiquement audit composé Cₒ sécrété par ladite cible Cᵢ, la liaison spécifique entre ledit ligand et ledit composé Cₒ générant un 2^{nd} signal distinct dudit 1^{er} signal, et
- la détection en temps réel dudit 2^{nd} signal et éventuellement la détection dudit 1^{er} signal,
une cible étant un élément sécréteur comprenant une ou plusieurs cellules identiques ou différentes, de type eucaryote ou procaryote.

Dans un tel procédé pour mesurer en temps réel la sécrétion d'un composé Cₒ par une cible Cᵢ, la cible Cᵢ peut ne pas être liée à la 1^{ère} surface et juste déposée ou sédimentée sur cette dernière. En variante, la 1^{ère} surface peut être fonctionnalisée par au moins une sonde se liant spécifiquement à ladite cible Cᵢ.

Ainsi, la présente invention propose un procédé pour mesurer en temps réel la sécrétion d'un composé Cₒ par une cible Cᵢ, ledit procédé comprenant ;
- la mise en culture, en milieu liquide, dans une chambre de culture d'une pluralité de cibles parmi lesquelles se trouve au moins une cible Cᵢ, ladite chambre de culture présentant :
   i) au moins une 1^{ère} surface fonctionnalisée par au moins une sonde se liant spécifiquement à ladite cible Cᵢ, la liaison spécifique entre ladite sonde et ladite cible Cᵢ générant un 1^{er} signal, et
   ii) au moins une 2^{nde} surface, différente de ladite 1^{ère} surface et non coplanaire avec ladite 1^{ère} surface, fonctionnalisée par au moins un ligand se liant spécifiquement audit composé Cₒ sécrété par ladite cible Cᵢ liée à ladite sonde, la liaison spécifique entre ledit ligand et ledit composé Cₒ générant un 2^{nd} signal distinct dudit 1^{er} signal, et
- la détection en temps réel dudit 2^{nd} signal et éventuellement la détection dudit 1^{er} signal,
une cible étant un élément sécréteur comprenant une ou plusieurs cellules identiques ou différentes, de type eucaryote ou procaryote.

Comme indiqué ci-dessus, on entend par « cible » un élément sécréteur comprenant une ou plusieurs cellules identiques ou différentes. Ainsi, une cible peut être (i) une cellule individuelle, (ii) un ensemble de cellules identiques tel qu'un groupement de cellules identiques encapsulées, (iii) un ensemble de cellules d'au moins deux types différents tel qu'un tissu éventuellement provenant d'une biopsie, d'une biopsie par forage/aspiration à l'aiguille fine (ou FNAB pour « Fine Needle Aspiration Biopsy »), d'une macrobiopsie ou d'une microbiopsie, un fragment de tissu éventuellement provenant d'une biopsie, d'une biopsie par forage/aspiration à l'aiguille fine (ou FNAB pour « Fine Needle Aspiration Biopsy »), d'une macrobiopsie, d'une microbiopsie ou encore (iv) un ensemble de cellules d'au moins deux types différents encapsulées. Dans le cadre de la présente invention, on entend par « tissu », aussi bien un tissu naturel qu'un tissu synthétique ou artificiel du type peau artificielle.

Ainsi, dans la présente invention, l'expression «une cible » peut être utilisée, de façon interchangeable, avec soit l'expression « une cellule », soit l'expression « des cellules ».

Dans le cadre de la présente invention, on entend par « cellule », aussi bien une cellule de type procaryote que de type eucaryote. Parmi les cellules eucaryotes, la cellule ou les cellules peu(ven)t être une levure telle qu'une levure du genre *Saccharomyces* ou *Candida,* une cellule de champignon, une cellule d'algue, une cellule végétale ou une cellule animale telle qu'une cellule de mammifères, de poissons ou d'insectes. Les cellules de mammifères peuvent notamment être des cellules tumorales, des lignées cellulaires immortalisées, des cellules somatiques ou germinales ou des cellules souches. Il peut s'agir de manière non exclusive de cellules endocrines, de cellules exocrines, de globules rouges, d'ostéoblastes, de cellules neuronales, de cellules nerveuses, de cellules épithéliales, d'hépatocytes, de cellules musculaires, des lymphocytes B, des lymphocytes T, de cellules caliciformes, de cellules chromaffines, de cellules de la granulosa, de cellules alpha ou bêta des îlots de Langerhans, de cellules progénitrices ou de cellules infectées par un agent infectieux tel qu'un virus.

Les cellules de type procaryote sont des bactéries qui peuvent être de type gram positif ou de type gram négatif, ou des archées. Parmi ces bactéries, on peut citer, à titre d'exemple et de façon non-exhaustive, les bactéries appartenant aux embranchements des spirochètes et des chlamydiae, les bactéries appartenant aux familles des entérobactéries, des streptococcacées, des microccacées, des légionelles, des mycobactéries, des bacillacées, des cyanobactéries et autres. Parmi les archées, on peut citer, à titre d'exemples et de façon non-exhaustive, les archées appartenant aux phyla des Crenarchaeotes et Euryarchaeotes.

Les cellules mises en oeuvre dans le cadre de la présente invention peuvent être obtenues à partir d'une culture cellulaire primaire ou d'une culture d'une lignée cellulaire, à partir d'un tissu ou d'une coupe de tissu éventuellement provenant d'une biopsie, d'une biopsie par forage/aspiration à l'aiguille fine (ou FNAB pour « Fine Needle Aspiration Biopsy »), d'une macrobiopsie ou d'une microbiopsie, ou à partir d'un échantillon issu d'un fluide tel qu'un fluide biologique ; un prélèvement dans un milieu de culture ou dans un réacteur de culture biologique comme une culture cellulaire ; un prélèvement dans une matrice alimentaire, de préférence dilué dans un tampon ; un prélèvement dans un réacteur chimique ; un prélèvement dans une station d'épuration ; un prélèvement dans une station de compostage ; de l'eau de ville, de rivière, d'étang, de lac, de mer, de piscines, de tours aéro-réfrigérées ou d'origine souterraine; un prélèvement à partir d'un effluent industriel liquide ; de l'eau usée provenant notamment d'élevages intensifs ou d'industries du domaine chimique, pharmaceutique ou cosmétique ou un prélèvement provenant d'une filtration d'air, ledit échantillon pouvant avoir subi différents traitements préalables comme une centrifugation, une concentration, une dilution, une encapsulation ....

Dans le cadre de la présente invention, on entend par « prélèvement » tout type de collecte d'échantillons, par exemple, par contact, raclage, forage, drainage, lavage, rinçage, aspiration, pompage, etc ...

Le fluide biologique est avantageusement choisi dans le groupe constitué par le sang tel que du sang entier ou du sang entier anti-coagulé, le plasma sanguin, la lymphe, la salive les larmes, le sperme, l'urine, les selles, le lait, le liquide céphalo-rachidien, le liquide interstitiel, un liquide synovial, un fluide isolé de moelle osseuse, un mucus ou fluide du tractus respiratoire, intestinal ou génito-urinaire, des extraits de tissus et des extraits d'organes. Ainsi, le fluide biologique peut être tout fluide naturellement sécrété ou excrété d'un corps humain ou animal ou tout fluide récupéré, à partir d'un corps humain ou animal, par toute technique connue de l'homme du métier telle qu'une extraction, un prélèvement, un drainage, ou un lavage. Les étapes de récupération et d'isolement voire d'encapsulation de ces différents fluides à partir du corps humain ou animal sont réalisées préalablement à la mise en oeuvre.

Dans le cadre du procédé selon la présente invention, la pluralité de cibles mises en culture en milieu liquide dans la chambre de culture correspond à un type de cibles tel que décrit ci-dessus (population homogène) ou, en variante, au moins deux types différents de telles cibles (population hétérogène). La cible Cᵢ, quant à elle, correspondant à l'une quelconque des cibles décrites ci-dessus.

Dans le cadre de la mise en culture dans un milieu liquide implique que les cibles mises en oeuvre se trouvent en suspension dans un milieu liquide adapté, propice à leur survie et à la sécrétion de biomolécules. A ce sujet, l'étape de mise en culture dans le procédé selon la présente invention s'entend dans des conditions adaptées ou convenables pour qu'au moins un composé soit sécrété par au moins une cible.

A noter toutefois que certaines de ces cibles, lorsqu'elles se fixent à une sonde les reconnaissant et fonctionnalisant une des surfaces de la chambre de culture, peuvent, de fait, se retrouver fixées à cette dernière. De même, dans la variante du procédé sans fonctionnalisation de la 1^{ère} surface par une sonde, certaines de ces cibles peuvent se déposer ou sédimenter sur une des surfaces de la chambre de culture.

De plus, les techniques et les conditions de culture cellulaire, notamment en milieu liquide, sont bien connues de l'homme du métier, qui saura définir, pour chaque type de cible et notamment chaque type cellulaire, le milieu nutritif, biologique ou synthétique, adéquat, la température de culture contrôlée optimale, par exemple 37°C, pour de nombreuses cellules de mammifères, ainsi que l'atmosphère nécessaire au maintien de la viabilité des cellules, par exemple 5% de CO₂. La durée de culture est également adaptable pour chaque type de cible et notamment chaque type cellulaire, en fonction de sa vitesse de sécrétion. Généralement, la durée de culture sera inférieure à 24 h. En outre, la culture pourra être stoppée dès que l'information recherchée, à savoir la détection d'un composé sécrété et la détermination de la cible sécrétrice, aura pu être obtenue.

La sonde apte à spécifiquement se lier à la cible Cᵢ est toute molécule capable de former avec la cible Cᵢ une paire de liaison, la sonde et la cible Cᵢ correspondant aux deux partenaires de cette paire de liaison. Les liaisons mises en oeuvre dans la liaison sonde-cible Cᵢ sont soit des liaisons non covalentes et de faible énergie telles que des liaisons hydrogène ou des liaisons de Van der Waals, soit des liaisons de forte énergie de type liaisons covalentes.

La sonde utilisée est donc dépendante de la cible Cᵢ à détecter. En fonction de cette cible Cᵢ, l'homme du métier saura, sans effort inventif, choisir la sonde la mieux adaptée. La sonde peut être toute 1^{ère} surface elle-même adaptée à la fixation des cibles telle qu'une surface en au moins un polymère adapté à la fixation des cibles. Ainsi, la sonde peut être choisie dans le groupe constitué par un peptide ; un oligopeptide ; une protéine ; une glycoprotéine ; un oligosaccharide ; un polysaccharide ; un glucide ; une lipoprotéine ; un lipide ; un phospholipide ; un agoniste ou un antagoniste d'un récepteur membranaire ; un anticorps polyclonal ou monoclonal ; un fragment d'anticorps tel qu'un fragment Fab, F(ab')₂, Fv, scFv, diabody ou un domaine hypervariable (ou CDR pour « Complementarity Determining Région ») ; une molécule nucléotidique ; un acide nucléique peptidique ; un aptamère tel qu'un aptamère ADN ou un aptamère ARN et un polymère adapté à la fixation de ces cibles tel qu'un polymère du type poly(N-isopropylacrylamide) (pNIPAM), un polymère du type pNIPAM-co-acrylamidophénylboronate (pNIPAM-co-APBA), un polypyrrole, une polylysine, un hydrocarbure aromatique polycyclique (PAH), un polyétherimide (PEI) ou un acide polyacrylique (PAA)....

L'expression « molécule nucléotidique » utilisée dans la présente invention est équivalente aux termes et expressions suivants : « acide nucléique », « polynucléotide », « séquence nucléotidique », « séquence polynucléotidique ». Par « molécule nucléotidique », on entend, dans le cadre de la présente invention, un polynucléotide régulateur ; un ADN, simple-brin ou double-brin, génomique, chloroplastique, plasmidique, mitochondrial, recombinant ou complémentaire ; une séquence faisant office d'aptamère ; une portion ou un fragment de ceux-ci.

A titre d'exemple illustratif et non limitatif, dans le cas où la cible Cᵢ est une cellule circulante, la sonde peut être choisie parmi les anticorps anti-CD (pour « Clusters of Differentiation »).

Le composé Cₒ sécrété dont on souhaite suivre, en temps réel, la production dans le procédé selon la présente invention peut être tout composé que peut sécréter une des cibles telles que précédemment décrites dans des conditions naturelles ou non. Dans ce dernier cas, la sécrétion du composé Cₒ peut être induite par des conditions particulières telles qu'un stress ou une infection.

Avantageusement, le composé Cₒ est ou comprend un élément choisi dans le groupe constitué par une protéine, un polypeptide, un peptide, un lipide, une glycoprotéine, un glycolipide, une lipoprotéine, un ion inorganique, une petite molécule organique comprenant de 1 à 100 atomes de carbone et un composé particulaire ou supramoléculaire, tel qu'une vésicule, un exosome, un organisme microbien comme, par exemple, un virus ou une particule microbienne comme, par exemple, une particule virale.

Plus particulièrement, le composé Cₒ est choisi dans le groupe constitué par une hormone, une pro-hormone, un neurotransmetteur, une cytokine, une chimiokine, une protéine de la matrice extra-cellulaire, une immunoglobuline, une toxine, un agent infectieux, tel qu'une bactérie, un virus, ou un parasite protozoaire, ou un constituant ou une production d'un agent infectieux, tel qu'une protéine ou une particule virale ou une toxine bactérienne.

Le ligand utilisé pour fonctionnaliser la 2^{nde} surface de la chambre de culture mise en oeuvre dans le cadre de la présente invention est toute molécule capable de former avec le composé Cₒ à détecter une paire de liaison, le composé Cₒ et le ligand correspondant aux deux partenaires de cette paire de liaison. Les liaisons mises en oeuvre dans la liaison ligand-composé Cₒ sont soit des liaisons non covalentes et de faible énergie telles que des liaisons hydrogène ou des liaisons de Van der Waals, soit des liaisons de forte énergie de type liaisons covalentes.

Le ligand utilisé est donc dépendant du composé Cₒ à détecter. En fonction de ce composé, l'homme du métier saura, sans effort inventif, choisir le ligand le mieux adapté. Il peut être choisi dans le groupe constitué par un peptide ; un oligopeptide ; une protéine ; une glycoprotéine ; un oligosaccharide ; un polysaccharide ; un glucide ; une lipoprotéine ; un lipide ; un phospholipide ; un anticorps polyclonal ou monoclonal ; un fragment d'anticorps tel qu'un fragment Fab, F(ab')₂, Fv, scFv, diabody ou un domaine hypervariable (ou CDR pour « Complementarity Determining Région ») ; un haptène ; une molécule nucléotidique telle que précédemment définie ; un acide nucléique peptidique ; un aptamère tel qu'un aptamère ADN ou un aptamère ARN, un polymère adapté à la fixation spécifique d'ions inorganiques, tel que ceux décrits par Lange *et al* (2008) [14] et un polymère adapté à la fixation de ces cibles tel qu'un polymère du type poly(N-isopropylacrylamide) (pNIPAM), un polymère du type pNIPAM-co-acrylamidophénylboronate (pNIPAM-co-APBA), un polypyrrole, une polylysine, un hydrocarbure aromatique polycyclique (PAH), un polyétherimide (PEI) ou un acide polyacrylique (PAA).

A titre d'exemple illustratif et non limitatif, un ligand utilisable dans le cadre du procédé selon la présente invention peut être tout anticorps utilisé dans les kits ELISA commerciaux.

Le procédé selon la présente invention dans lequel la 1^{ère} surface n'est pas fonctionnalisée par au moins une sonde reconnaissant au moins une cible Ci se base non seulement sur l'utilisation d'une paire de liaison particulières à savoir la paire de liaison ligand-composé Cₒ mais aussi sur une disposition spatiale des cibles et ligands, particulière dans la chambre de culture.

En variante, le procédé selon la présente invention dans lequel la 1^{ère} surface est fonctionnalisée par au moins une sonde reconnaissant au moins une cible Ci se base non seulement sur l'utilisation de deux paires de liaison particulières à savoir, d'une part, la paire de liaison sonde-cible Cᵢ et, d'autre part, la paire de liaison ligand-composé Cₒ mais aussi sur une disposition spatiale des ligands et sondes, particulière dans la chambre de culture.

En effet, dans les procédés selon la présente invention, la 2^{nde} surface fonctionnalisée par des ligands est différente de la 1^{ère} surface éventuellement fonctionnalisée par des sondes et non coplanaire avec cette 1^{ère} surface. Cette caractéristique exclut explicitement que la 1^{ère} surface et la 2^{nde} surface se trouvent conjointement au niveau d'une même surface d'un support solide non physiquement structuré. En d'autres termes, la 1^{ère} surface et la 2^{nde} surface ne correspondent pas à des régions distinctes du type spots réparties sur une des surfaces d'un support solide non physiquement structuré. Ainsi, la 1^{ère} et la 2^{nde} surfaces sont physiquement distinctes.

Toutefois, la 1^{ère} surface et la 2^{nde} surface se trouvent à une distance telle que, d'une part, le signal émis par la présence de la cible Cᵢ sur la 1^{ère} surface ou par la liaison spécifique entre la sonde et la cible Cᵢ (désigné dans la présente par 1^{er} signal) se distingue du signal émis par la liaison spécifique entre ledit ligand et ledit composé Cₒ (désigné dans la présente par 2^{nd} signal) et que, d'autre part, c'est le composé Cₒ sécrété par une cible Cᵢ présente sur la 1^{ère} surface et éventuellement liée à une sonde spécifique correspondante, qui se fixe à un ligand donné. En d'autres termes, le 1^{er} signal et le 2^{nd} signal se distinguent l'un de l'autre i.e. n'interfèrent pas l'un avec l'autre et/ou ne se superposent pas. En variante, au moins l'un des signaux est indépendant permettant ainsi de différencier l'origine des signaux.

De plus, le procédé et le dispositif selon la présente invention mettent en oeuvre une organisation spatiale spécifique de la ou des cible(s) Cᵢ et du ou des ligand(s) spécifique(s) du composé Cₒ, dans le cas où la 1^{ère} surface est non fonctionnalisée. En variante, le procédé avec fonctionnalisation de la 1^{ère} surface et le dispositif selon la présente invention mettent en oeuvre une organisation spatiale spécifique du ou des sonde(s) spécifique(s) de la cible Cᵢ et de ou des ligand(s) spécifique(s) du composé Cₒ. Il existe une proximité entre ces cibles et ces ligands ou entre ces sondes et ces ligands de façon à garantir que le composé Cₒ sécrété par une cible Cᵢ éventuellement liée à une sonde spécifique se fixe à un ligand donné. Ainsi, la 1^{ère} surface et la 2^{nde} surface sont organisées spatialement de manière à pouvoir attribuer avec certitude l'origine cellulaire du type de sécrétion détectée lorsque les molécules sécrétées diffusent dans l'environnement immédiat des cibles. Plus particulièrement, la cible et le ligand sont distants l'un de l'autre de 100 nm à 500 µm et avantageusement d'au moins 300 nm. Lorsque la 1^{ère} surface comprend plusieurs cibles Cᵢ et lorsque la 2^{nde} surface comprend plusieurs ligands spécifiques du composé Cₒ, chaque sonde est distante de chaque cible d'au moins 100 nm. En variante, la sonde et le ligand sont distants l'un de l'autre de 100 nm à 500 µm et avantageusement d'au moins 300 nm. Lorsque la 1^{ère} surface comprend plusieurs sondes spécifiques de la cible Cᵢ et lorsque la 2^{nde} surface comprend plusieurs ligands spécifiques du composé Cₒ, chaque sonde est distante de chaque ligand d'au moins 100 nm.

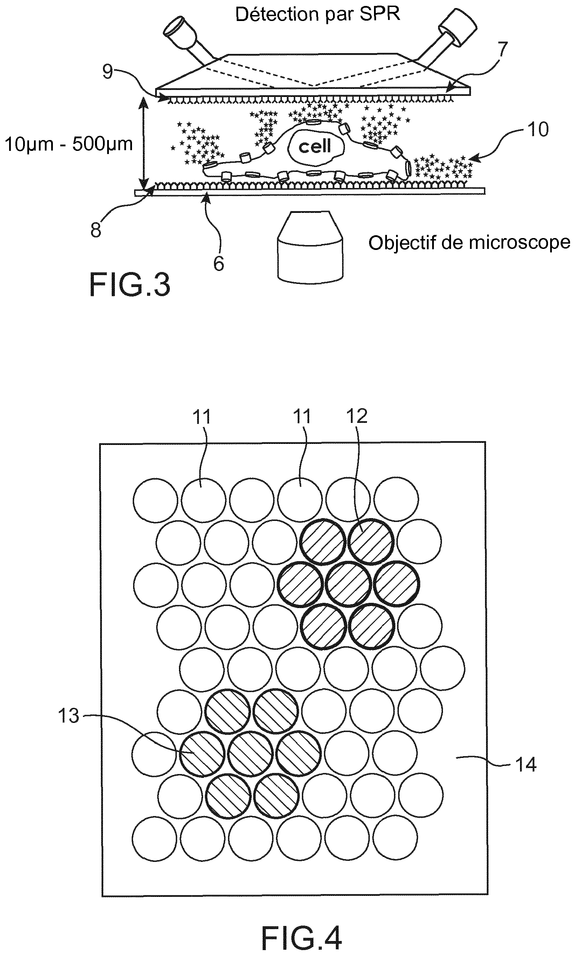

Dans une 1^{ère} forme de mise en oeuvre, la 1^{ère} surface et la 2^{nde} surface appartiennent à deux supports solides différents présents dans la chambre de culture. Avantageusement, ces deux supports solides sont placés en vis-à-vis l'un de l'autre et sont séparés par un espace, typiquement rempli de milieu de culture tel que précédemment défini, de 10 à 500 µm. A titre d'exemples, ces deux supports solides peuvent être deux parois de la chambre de culture, placées en vis-à-vis l'une de l'autre. La Figure 3 ci-après concerne cette 1^{ère} forme de mise en oeuvre.

Dans cette dernière, les deux supports solides peuvent être de nature identique ou différente. La nature d'un des deux supports solides est fonction de la technique de détection utilisée pour détecter le 1^{er} signal, alors que celle de l'autre support solide est fonction de la technique de détection utilisée pour détecter le 2^{nd} signal.

Dans cette configuration, plusieurs faces ou supports solides sont soit aptes à recevoir une cible, soit fonctionnalisées par un type de ligand spécifique d'une sécrétion. La détection de la présence des cibles sur certain(e)s faces ou supports solides et des interactions ligand-composé sur d'autres faces ou supports solides peut se faire soit par la même technique, soit par des techniques différentes.

En variante, dans cette configuration, plusieurs faces ou supports solides sont fonctionnalisées soit par un type de sonde spécifique d'une cible, soit par un type de ligand spécifique d'une sécrétion. La détection des interactions sur ces faces ou supports solides peut se faire soit par la même technique, soit par des techniques différentes.

Dans cette 1^{ère} forme de mise en oeuvre, il est aussi possible de fonctionnaliser la 2^{nde} surface, en regard de la 1^{ère} surface fonctionnalisée par des sondes spécifiques d'une cible Cᵢ, avec plusieurs types de ligands spécifiques de différents composés Cₒ. Chaque type de ligands est avantageusement regroupé sous forme d'une région discrète d'affinité i.e. présentant une affinité pour un composé Cₒ donné.

Dans une 2^{nde} forme de mise en oeuvre, la 1^{ère} surface et la 2^{nde} surface dépendent d'un même support solide tel que le fond de la chambre de culture. Cette 2^{nde} forme de mise en oeuvre admet différentes alternatives.

Dans cette 2^{nde} forme de mise en oeuvre, le support solide est physiquement structuré. Par « physiquement structuré », on entend un support présentant une structuration physique impliquant des éléments en relief du type microstructures, plots, piliers, puits ou toute autre forme géométrique avantageusement choisie telle que des particules ou billes dont la surface est apte à recevoir des cibles ou présente une affinité pour certaines cibles, et déposées sur une surface ayant une affinité pour des sécrétions.

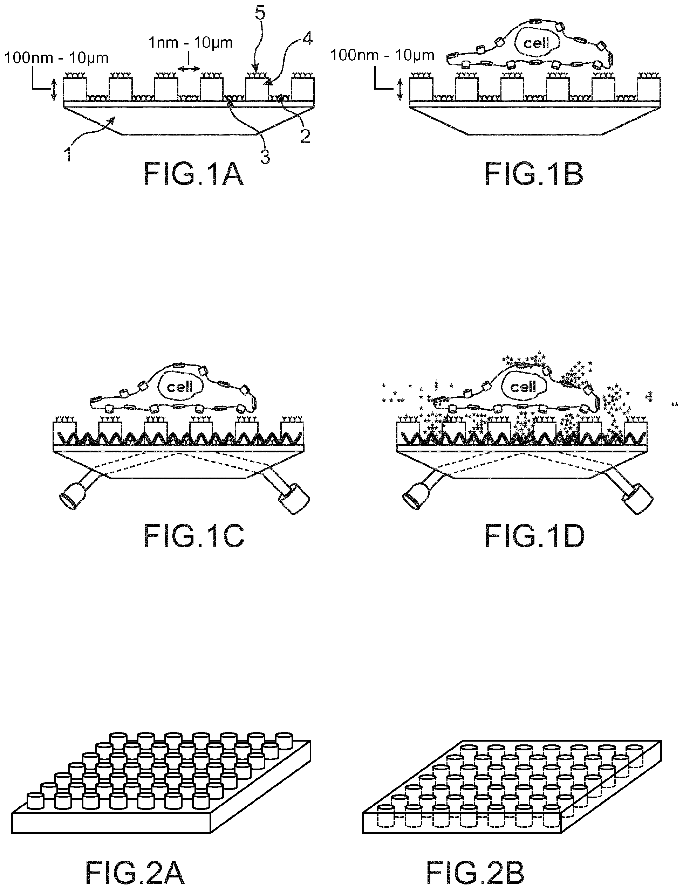

Les Figures 1 et 2 ci-après concernent une 1^{ère} alternative de cette 2^{nde} forme de mise en oeuvre, cette alternative portant sur des microstructures, plots, piliers et puits.

Dans cette alternative, la structuration physique permet la création de zones en profondeur (i.e. fonds des puits ou zones du support solide entourant les microstructures, plots ou piliers) et de zones en hauteur (i.e. bord supérieur des puits ou extrémité distale des microstructures, plots ou piliers, l'extrémité proximale étant en contact avec le support solide). La surface des zones en profondeur constitue une ou plusieurs 2^{nde(s)} surface(s) telle(s) que précédemment définie(s) et est donc fonctionnalisée par des ligands se liant spécifiquement à au moins un composé Cₒ. De même, les zones en hauteur correspondent à une ou plusieurs 1^{ère(s)} surface(s) telle(s) que précédemment définie(s), ces zones en hauteur pouvant éventuellement être fonctionnalisée(s) par des sondes se liant spécifiquement à au moins une cible Cᵢ.

Avantageusement, ces éléments en relief du type microstructures présentent une hauteur comprise entre 100 nm et 10 µm et sont avantageusement espacées les unes des autres de 1 nm à 10 µm, ledit espacement pouvant être constant ou variable.

Dans cette 1^{ère} alternative de la 2^{nde} forme de mise en oeuvre, le support solide et la ou les microstructure(s) peuvent être de nature identique ou différente. La nature de la ou des microstructure(s) est fonction de la technique de détection utilisée pour détecter le 1^{er} signal, alors que celle du support solide est fonction de la technique de détection utilisée pour détecter le 2^{nd} signal.

Cette alternative peut permettre de réaliser un procédé et un dispositif multiplexé. En effet, une ou plusieurs microstructures proches les unes des autres peuvent être fonctionnalisées par une sonde spécifique d'un type de cible et notamment d'un type cellulaire, définissant ainsi une zone d'affinité pour un tel type de cible ou cellulaire et le support solide peut présenter différentes microstructures définissant différentes zones d'affinité, chacune étant spécifique d'un type de cible ou cellulaire donné. De même, la surface du support solide peut présenter différentes régions d'affinité pour différents composés Cₒ.

Dans une 2^{nde} alternative de cette 2^{nde} forme de mise en oeuvre, une ou plusieurs particule(s) sont disposées sur un support solide présentant au moins une 2^{nde} surface telle que précédemment définie, tout ou partie de la surface d'au moins une particule correspondant à une 1^{ère} surface telle que précédemment définie.

Par « particule », on entend une particule solide, sphérique ou sensiblement sphérique notamment du type bille, présentant un diamètre compris entre 500 nm et 50 µm, ladite particule étant éventuellement poreuse. Lorsque plusieurs particules sont mises en oeuvre, il peut être avantageux d'utiliser des particules monodisperses ou des mélanges de différents types de particules, chaque type de particules étant monodisperse et ce, afin d'obtenir un dépôt homogène et, par là-même, un 1^{er} signal tel que précédemment défini, homogène.

Dans cette alternative, les particules mises en oeuvre ne sont pas des solides de Platon avec remplissage complet de l'espace entre les particules. Ainsi, les espaces vides entre les particules créent des pores qui permettent aux composés sécrétés par les cibles déposées ou fixées à la surface de telles particules de cheminer jusqu'aux ligands fonctionnalisant la surface du support solide sous-jacent. En d'autres termes, cette alternative correspond à un assemblage de particules solides dont l'organisation crée des pores. Ainsi les particules solides assemblées forment une couche poreuse permettant de séparer ou filtrer les cibles des composés à détecter.

Par conséquent, dans cette alternative, plusieurs particules solides sont disposées sur un support solide présentant au moins une 2^{nde} surface telle que précédemment définie, tout ou partie de la surface d'au moins une de ces particules solides correspondant à une 1^{ère} surface telle que précédemment définie et ces particules solides formant une couche poreuse.

Avantageusement, la taille des pores est inférieure au 1/3 du diamètre des particules. Plus particulièrement, la taille des pores est comprise entre 100 et 500 nm moyennant quoi le cheminement des composés sécrétés jusqu'aux ligands fonctionnalisant la surface du support solide sous-jacent est possible. L'utilisation de particules dont l'organisation crée des pores permet, entre autre, d'augmenter la sensibilité du procédé et du dispositif en réduisant l'espace occupé par les particules qui peuvent en outre être poreuses. De plus, lorsque les particules mises en oeuvre sont poreuses, les composés peuvent également cheminer via la porosité ouverte de ces dernières.

De plus, dans cette 2^{nde} alternative de la 2^{nde} forme de mise en oeuvre, il est possible de mettre en oeuvre des particules présentant d'un côté des fonctions permettant l'accrochage à la surface du support solide sous-jacent et de l'autre côté des sondes telles que précédemment définies. De telles particules à deux faces distinctes sont connues sous le nom de particules de Janus.

Dans cette alternative, il est possible d'assembler des particules de nature identique ou différente et présentant des fonctionnalisations différentes et de contrôler leur position sur la surface du support solide sous-jacent créant ainsi des zones d'affinité, ce dernier présentant également des régions discrètes fonctionnalisées par des ligands spécifiques de différents composés Cₒ sécrétés (régions d'affinité). Ainsi, il est possible, dès le dépôt d'obtenir des surfaces ayant des motifs sensibilisés à détecter des substances et cellules différentes. La possibilité de contrôler la localisation spatiale de différentes fonctions sur la surface dès l'étape de dépôt est un net avantage pour la commercialisation d'un dispositif de détection multi-composants. Cet avantage peut énormément faciliter la fonctionnalisation biochimique nécessaire pour la spécificité du dispositif et ce, déjà simplement par la réduction du nombre d'étapes de préparation.

Dans un tel assemblage, les particules peuvent se présenter sous forme d'une monocouche ou sous forme d'une superposition de monocouches. Avantageusement, les particules se présentent sous forme d'une monocouche du type film compact de particules.

L'homme du métier connaît différentes techniques permettant de réaliser des dépôts de film compact de particules, les plus connues étant :
- la méthode Langmuir-Blodgett qui met en oeuvre un liquide porteur (par exemple de l'eau) dans lequel est préalablement immergé en position verticale le support solide i.e. le substrat « cible » sur lequel doit être reportée la monocouche de particules. Les particules sont dispensées à la surface du liquide sur laquelle elles se dispersent. Une barrière mécanique est alors mise en mouvement pour réduire progressivement la surface occupée par les particules afin de les mettre en compression. Lorsque le film compact est formé, le substrat est mis en mouvement pour déposer par capillarité le film à sa surface. La barrière doit accompagner ce mouvement de tirage afin de conserver la mise en compression des particules [15],

- par enduction centrifuge, plus connue sous le terme anglais de « spin coating » [16] ;
- par la technique de trempage-retrait, plus connue sous le terme anglais de « dip-coating », technique connue de l'homme de l'art ; et
- et plus particulièrement par un procédé original du CEA [17] explicité dans la partie expérimentale ci-après.

Cette 2^{nde} alternative de la 2^{nde} forme de mise en oeuvre présente de nombreux avantages à savoir (i) une géométrie quasi-idéale avec une minimisation du contact entre le support solide et les particules conférant une architecture optimale pour que le dispositif utilisant notamment une détection par résonance de plasmon de surface (SPR) ne perde pas de sensibilité ; (ii) un contrôle parfait de l'assemblage et de la taille des particules ce qui permet d'obtenir une reproductibilité du système et une technologie de dépôt rapide, bas coût, effective sur des objets volumiques de géométrie complexe ; et (iii) des interstices réguliers et connus, plus confortables que pour une porosité stochastique (du type sol gel).

De plus, un tel dispositif est suffisamment robuste pour être manipulé dans différents solvants tels que, par exemple, éthanol, acétone, toluène ou eau pour sa fonctionnalisation chimique, et sous flux hydrodynamique pour le rinçage et son utilisation en microfluidique. Enfin, il est biocompatible permettant de réaliser des études biologiques *in vitro.*

Quelles que soient la forme de mise en oeuvre ou l'alternative considérées, les surfaces mises en oeuvre peuvent présenter différentes zones ou régions d'affinité telles que précédemment définies. Le nombre de sondes ou de ligands différents immobilisés sur chacune de ces zones ou régions peut aller, par exemple, de 1 à 1000 et notamment de 1 à 500. La taille des zones ou régions peut être nanométrique, micrométrique, millimétrique ou centimétrique.

De même, quelles que soient la forme de mise en oeuvre ou l'alternative considérées, les supports solides mis en oeuvre, les éléments en relief, les particules, la 1^{ère} surface et/ou la 2^{nde} surface tel(le)s que précédemment défini(e)s peuvent être des supports solides, des éléments en relief, des particules ou des surfaces en matériau organique, en matériau inorganique ou en un mélange d'au moins un matériau organique et d'au moins un matériau inorganique.

A titre d'exemples de mélange, on peut envisager le cas où au moins une surface choisie entre ladite 1^{ère} surface et ladite 2^{nde} surface est en un matériau organique, l'autre surface étant en un matériau inorganique.

Avantageusement, le matériau inorganique est choisi dans le groupe constitué par les verres, les quartz, les céramiques (par exemple, de type oxyde), les métaux (par exemple, platine, aluminium, chrome, cuivre, zinc, argent, nickel, étain ou or), les métalloïdes (par exemple, silicium ou silicium oxydé), les carbones allotropiques (par exemple, carbone vitreux, graphite, graphène, nanostructure carbonée du type nanoparticule ou nanotube de carbone, ou diamant) et leurs mélanges.

Lorsque le matériau des supports solides, des éléments en relief, de la 1^{ère} surface et/ou de la 2^{nde} surface est organique, il correspond avantageusement à un matériau polymère ou une résine organique comme, par exemple, de l'agarose, un polyamide (type nylon), un polycarbonate, un polyéthylène glycol, un fluoropolymère, un acrylate, un siloxane (polydiméthylsiloxane ; PDMS), un copolymère oléfinique cyclique (COC), un polyéther éther cétone (PEEK) ou de la nitro-cellulose.

La fonctionnalisation par une sonde et un ligand tels que précédemment définis respectivement de la 1^{ère} surface et la 2^{nde} surface telles que précédemment définies peut être effectuée par toute technique adaptée permettant la fixation d'un composé sur un support solide. On peut en particulier envisager une simple adsorption, des liaisons ioniques, des liaisons hydrogène, des interactions électrostatiques, des interactions hydrophobes, des liaisons de Van der Waals ou un greffage covalent.

Dans une forme de mise en oeuvre particulière de la présente invention, la 1^{ère} ou la 2^{nde} surface présente des groupements fonctionnels grâce auxquels la (ou les) sonde(s) ou le (ou les) ligand(s) est(sont) capable(s) de s'immobiliser. De façon avantageuse, ces groupements fonctionnels sont choisis parmi les groupements carboxyliques, des entités radicalaires, les fonctions alcools, amines, amides, époxy ou thiols. Ces groupements peuvent être intrinsèques à la nature du matériau de la 1^{ère} ou de la 2^{nde} surface. De façon alternative, ces derniers peuvent être obtenus par nettoyage de ladite 1^{ère} ou 2^{nde} surface par l'intermédiaire d'au moins un solvant, détergent, rayonnement ou plasma d'oxygène ou tout autre procédé permettant la formation de groupements fonctionnels tels que précédemment définis.

Dans une première variante de la présente invention, la (ou les) sonde(s) peu(ven)t être immobilisée(s) de façon directe au niveau de la 1^{ère} surface fonctionnalisée ou non. Un support solide « coaté » avec une protéine est un exemple d'immobilisation directe.

Dans une seconde variante de la présente invention, la (ou les) sonde(s) peu(ven)t être immobilisée(s) de façon indirecte au niveau de la 1^{ère} surface fonctionnalisée ou non. Cette immobilisation indirecte fait intervenir un bras espaceur (ou agent de jonction) lié, d'une part, à la 1^{ère} surface et, d'autre part, à une sonde. L'homme du métier connaît différents exemples de tels bras espaceurs. De façon non exhaustive, peuvent être cités, en tant que bras espaceurs susceptibles d'être mis en oeuvre dans le cadre de la présente invention, le 1,6 diaminohexane, l'acide 6-aminohexanoïque, un silane, un groupe succinimide, un époxyde, l'acide UDP-glucuronique, des chaînes alkyles linéaires ou ramifiées de 1 à 20 atomes de carbone, du polyéthylène glycol, du glutaraldéhyde, etc... Tout ce qui vient d'être décrit sur la 1^{ère} surface et les sondes s'applique *mutatis mutandis* à la 2^{nde} surface et aux ligands.

Cette immobilisation indirecte appliquée au dispositif comprenant des particules solides disposées sur un support solide implique que ledit support solide soit fonctionnalisé chimiquement pour permettre l'assemblage covalent ou non covalent desdites particules solides.

A titre d'exemples illustratifs, on peut plus particulièrement citer les réactifs silanes mis en oeuvre pour le greffage de ligand(s) ou de sonde(s) sur du verre, la complexation de produits thiolés sur des surfaces d'or et l'immobilisation de ligand(s) ou de sonde(s) dans des matrices polymères.

A noter que l'immobilisation préalable sur le support comprenant la 1^{ère} surface d'un bras espaceur peut être non seulement être mise en oeuvre pour permettre la fonctionnalisation par un ou plusieurs ligand(s) mais aussi pour permettre l'accrochage des particules solides telles que précédemment définies sur ce support. Un exemple particulier d'un tel bras espaceur est une monocouche auto-assemblée (SAM) de mercaptopropyltriéthoxysilane ou mercaptopropyltriméthoxysilane (MPTS) notamment utilisée sur des surfaces d'or.

Les liaisons mises en oeuvre au cours d'une immobilisation directe ou indirecte peuvent être toutes liaisons connues de l'homme du métier et notamment des liaisons covalentes, des liaisons ioniques, des liaisons hydrogène, des interactions électrostatiques, des interactions hydrophobes, des liaisons de Van der Waals, une adsorption, etc...

Dans le cadre de la présente invention, le 1^{er} (ou le 2^{nd}) signal est produit lors et/ou à partir du dépôt de la cible Cᵢ sur ladite 1^{ère} surface ou lors et/ou à partir de la liaison de la cible Cᵢ à la sonde (ou du composé Cₒ au ligand) et est mesurable par un élément de mesure du signal. La détection ou mesure d'un signal désigne la détermination de la présence ou de l'absence du 1^{er} (ou du 2^{nd}) signal, et/ou sa quantification. L'expression en « temps réel » signifie que le 2^{nd} signal est produit et mesuré essentiellement au moment où la liaison du composé Cₒ avec le ligand a lieu.

Comme déjà explicité, l'organisation spatiale mise en oeuvre entre la 1^{ère} surface et la 2^{nde} surface permettent de garantir que, lorsqu'un composé Cₒ est détecté sur la 2^{nde} surface, la cible sécrétrice se trouve au niveau de la 1^{ère} surface et éventuellement liée à une sonde. La mesure du 1^{er} signal n'est donc pas obligatoire mais constitue une mesure de contrôle à des fins vérificatives.

La mesure du 2^{nd} signal selon l'invention est effectuée directement. Ainsi, le signal ne nécessite pas la médiation de molécules présentes dans la chambre de culture ou ajoutées à la chambre de culture, autres que le composé Cₒ et le ligand, pour être produit. A titre d'exemple, le signal selon l'invention ne provient pas d'une sonde d'oxydoréduction, ou de la liaison additionnelle d'un marqueur spécifique du composé Cₒ, tel qu'un anticorps marqué, sur un composé Cₒ déjà fixé par le ligand. Tout ce qui vient d'être explicité sur la mesure du 2^{nd} signal s'applique *mutatis mutandis* à la mesure du 1^{er} signal.

Avantageusement, toute technique de détection en temps réel et notamment toute technique de suivi de réaction d'interaction en temps réel connues de l'homme du métier sont utilisables pour mesurer le 2^{nd} signal. Plus particulièrement, cette technique de détection est choisie dans le groupe constitué par la résonance des plasmons de surface en mode monopoint, la résonance des plasmons de surface en imagerie, une technique optique en champ proche et la mesure d'impédance. Des compléments d'informations sur les techniques impliquant une résonance de plasmons de surface peuvent être obtenus dans la demande de brevet US 2013/137085 **[12].**

En ce qui concerne la détection du 1^{er} signal, cette dernière fait avantageusement intervenir une technique optique telle que, par exemple, la microscopie optique, la microscopie optique en mode guidé ou la microscopie de fluorescence. Toutefois, d'autres modes d'imagerie optique peuvent également être mises en oeuvre pour détecter le 1^{er} signal.

Il convient de noter que aussi bien la détection du 1^{er} signal que celle du 2^{nd} signal nécessitent au moins un élément de transduction de ce 1^{er} signal ou de ce 2^{nd} signal susceptible de transmettre en temps réel le 1^{er} ou le 2^{nd} signal produit par la présence d'un objet ou par la liaison d'un type d'objet (cible Cᵢ ou composé Cₒ) sur la zone affine spécifique i.e. fonctionnalisée par un ligand spécifique ou éventuellement par une sonde spécifique. Ainsi, un élément de transduction du signal (mesure du seul 2^{nd} signal) ou deux éléments de transduction du signal, identiques ou différents, sont mis en oeuvre dans le cadre de la présente invention. Un élément de transduction du signal est tout élément permettant une détection en temps réel en s'affranchissant de la présence de médiateurs/révélateurs quel qu'ils soient. Il est avantageusement choisi dans le groupe constitué par un analyseur d'impédance, une poutre résonante et un système de lecture optique tel qu'un imageur de résonance de plasmons de surface, un système de lecture optique par microscopie optique ou un système de lecture optique par microscopie fluorescente.

La présente invention concerne également certains des dispositifs susceptibles d'être mis en oeuvre dans un procédé tel que précédemment défini. Ces dispositifs ont été précédemment décrits et correspondent, plus particulièrement,
- d'une part, au dispositif comprenant une chambre de culture dans laquelle la 1^{ère} surface et la 2^{nde} surface appartiennent à deux supports solides différents et,
- d'autre part, au dispositif tel que précédemment défini, comprenant une chambre de culture dans laquelle une ou plusieurs particule(s) sont disposées sur un support solide présentant au moins une 2^{nde} surface telle que précédemment définie, tout ou partie de la surface d'au moins une particule correspondant à une 1^{ère} surface telle que précédemment définie.

Le procédé et les dispositifs selon la présente invention présentent de nombreuses applications dans lesquelles il est nécessaire de mesure la sécrétion ou la libération d'un ou plusieurs composés par une ou plusieurs cibles cellulaires sécrétrices données.

Un des champs généraux d'application du procédé et des dispositifs selon l'invention porte sur des technologies pour la santé, et plus particulièrement pour des applications tournées vers l'analyse de fluides biologiques tels que précédemment définis, et également pour des applications diagnostiques. A titre d'exemples illustratifs et non limitatifs de telles applications, on peut citer :
- l'évaluation de la couverture vaccinale (test HBV, test Grippe et test Tuberculose) ;
- la caractérisation d'états immuno-déprimés (congénitaux ou acquis comme le SIDA ou résultant de traitements comme dans les greffes d'organes) ;
- la caractérisation de maladies auto-immunes (diabète, lupus, sclérose en plaques etc) et
- des tests préliminaires de compatibilité de greffons.

Un autre champ d'application du procédé et des dispositifs selon la présente invention consiste en leur utilisation dans l'industrie pharmaceutique, notamment dans les projets de criblage de molécules à potentiel immuno-modulateur, soit stimulant comme dans le cadre de vaccins, soit déprimant comme dans le cadre d'anti-inflammatoires. Puisqu'il s'agit dans ces cas d'identifier des molécules susceptibles de déclencher une réaction immunitaire contrôlée, la présente invention semble particulièrement adaptée pour détecter rapidement et de manière plus précise (réponse cinétique de cellules individuelles) et rapide toute molécule régulant le système immunitaire. A cet effet, la molécule à tester est présente dans le milieu de culture et la mesure de la sécrétion d'un composé Cₒ par une cible sécrétrice Cᵢ obtenue en présence de ladite molécule à tester est comparée à la sécrétion du même composé Cₒ par une même cible sécrétrice Cᵢ obtenue en absence de cette molécule.

Une autre application plus académique du procédé et des dispositifs selon l'invention peut consister à étudier l'influence d'une condition donnée sur une cible Cᵢ et notamment sur la sécrétion du composé Cₒ sécrété par cette dernière.

Avantageusement, la condition dont on cherche à déterminer l'influence sur la cible Cᵢ et sur sa sécrétion est une condition physique, chimique ou biologique.

Par « condition physique », on entend une condition physique qui modifie l'environnement dans lequel se trouve la cible Cᵢ telle qu'une condition thermique (modification de la température du milieu de culture), une condition électrique ou électromagnétique (environnement et donc cible Cᵢ soumis à une stimulation électrique ou une onde électromagnétique), une condition mécanique ou une condition radioactive.

Par « condition chimique » on entend une condition chimique qui modifie l'environnement dans lequel se trouve la cible Cᵢ telle que l'adjonction d'un composé à tester dans le milieu de culture et/ou la modification de sa concentration, la modification de la nature et/ou de la concentration des ions contenus dans ledit milieu de culture.

Par « condition biologique », on entend une condition de nature biologique qui modifie l'environnement dans lequel se trouve la cible Cᵢ telle que la présence de cellules appartenant à un autre type cellulaire, bactéries, archées, parasites, champignons, levures ou virus, ou la présence de biomolécules (chimiokines, fragments antigéniques, changement de milieu de culture..).

Dans cette application aussi, la mesure de la sécrétion d'un composé Cₒ par une cible sécrétrice Cᵢ est comparée en présence et en absence de la condition à tester.

L'invention sera mieux comprise à la lecture des figures et exemples qui suivent. Ceux-ci n'ont pas pour but de limiter l'invention dans ses applications, il ne s'agit que d'illustrer ici les possibilités offertes par le procédé et les dispositifs de l'invention.

### BRÈVE DESCRIPTION DES DESSINS

La Figure 1 présente un exemple de mise en oeuvre avec des sondes et des ligands déposés sur le même support solide. Le support solide est structuré (Figure 1A) de manière à faire apparaître des régions hautes fonctionnalisées par des sondes spécifiques des types cellulaires (régions en contact avec les cellules) (Figure 1B) ainsi que des régions basses (ici au fond des puits) pour capturer à l'aide de sondes spécifiques les molécules sécrétées et ainsi les détecter par une onde évanescente (Figure 1C) lors de l'activation du phénomène de sécrétion cellulaire (Figure 1D).
La Figure 2 présente deux modes de réalisation des domaines structurés à la surface d'un même support solide, soit par des plots ce qui correspond à une configuration identique à celle de la Figure 1 (Figure 2A), soit par des puits (Figure 2B).
La Figure 3 présente un exemple de montage d'un dispositif muni de deux faces fonctionnalisées, l'une (matériau en bas) dédiée à la capture de cellules sur des régions d'affinité spécifiques et l'autre (matériau en haut) propice à la détection de molécules sécrétées (et diffusant depuis les cellules immobilisées à proximité) par des ligands dont la liaison à leur cible est suivie par résonance plasmonique de surface.
La Figure 4 présente le dépôt schématisé de billes préfonctionnalisées différemment pour une immobilisation de cellules différenciées.

### EXPOSÉ DÉTAILLÉ DE MODES DE RÉALISATION PARTICULIERS

### 1. Dispositifs mis en oeuvre dans le procédé selon l'invention.

Ces dispositifs présentent une face structurée. En effet, afin de distinguer le signal dû aux cibles et celui dû aux sécrétions, certains travaux à la base de la présente invention ont visé à structurer la surface d'analyse afin d'en éloigner les cibles cellulaires. Notons que la dimension des cellules (1 à 10 µm) est généralement supérieure de plusieurs ordres de grandeurs aux sécrétions (1 à 100 nm).

Plus particulièrement, la lecture du présent biocapteur se faisant à l'aide d'imagerie de résonance de plasmon de surface (SPR) pour les sécrétions et par un autre mode comme la microscopie optique pour les cellules, le dispositif **1** contient une structuration de surface comme suit :
- des régions « en profondeur » **2** du dispositif constituant une ou plusieurs 2^{ème(s)} surface(s) telle(s) que précédemment définie(s) sont fonctionnalisées par des ligands **3** spécifiques des sécrétions. Cela peut être par exemple des anticorps anti-cytokines déposés sur une surface d'or, dont la complexation avec l'antigène i.e. la cytokine produit un signal en imagerie SPR ; et
- des régions « en hauteur » **4,** c'est à dire à une altitude supérieure à 100 nm afin de se situer hors champ de la propagation de l'onde évanescente générée par les plasmons de surface. Ces domaines correspondant à une ou plusieurs 1^{ère(s)} surface(s) telle(s) que précédemment définie(s) sont fonctionnalisés par des sondes **5** spécifiques de différents types cellulaires. La taille de ces domaines est supérieure à la taille d'une cellule individuelle (soit une dizaine de microns de diamètre environ), et peut atteindre plusieurs millimètres carrés, voire un centimètre carré.

Afin de dissocier les signaux détectés par SPR dus soit à la capture des cellules, soit à celle des biomolécules sécrétées, la mise en oeuvre d'un dispositif dont une face sensible du biocapteur est structurée peut être envisagée. La propagation de l'onde évanescente produite par la résonance des plasmons se produit sur une centaine de nanomètres au-dessus du film métallique. Plusieurs régions fonctionnalisées, à différentes « altitudes » au-dessus du film sensible du biocapteur peuvent être fabriquées pour permettre, par exemple, la capture spécifique de cellules en hauteur (i.e. hors champ de la détection SPR), et la capture des sécrétions à basse altitude (dans le champ de la détection SPR) selon la Figure 1.

Les supports contenant les partenaires de liaison des cellules et des composés sécrétés peuvent être soit des piliers dont les sommets sont fonctionnalisés par des sondes se liant spécifiquement à un ou plusieurs type(s) cellulaire(s) et les espaces entre les piliers par des ligands se liant spécifiquement à un ou plusieur(s) type(s) de composé(s) (Figure 2A), soit une surface plane fonctionnalisée par des sondes se liant spécifiquement à un ou plusieurs type(s) cellulaire(s), et perforée de part en part pour permettre une fonctionnalisation des puits ainsi formés par des ligands se liant spécifiquement à un ou plusieur(s) type(s) de composé(s) (Figure 2B).

Toutefois, les techniques mises en oeuvre pour cette structuration et les dispositifs finalement obtenus peuvent, dans certains cas, présenter un ou plusieur(s) inconvénient(s) choisi(s) parmi une réalisation très lente et donc impossible à industrialiser, une perte de sensibilité, une fragilité du dispositif notamment dans les milieux biologiques et des matériaux difficiles à caractériser.

### 2. Dispositifs selon la présente invention.

### 2.1. Dispositif contenant deux faces fonctionnalisées.

Ce montage contient deux faces **6** et **7,** fonctionnalisées et placées en vis-à-vis l'une de l'autre, séparées par un espaceur de 10 à 500 µm. Cet espaceur rempli de milieu de culture permet la diffusion des sécrétions depuis les cibles cellulaires capturées sur une face vers les ligands immobilisés sur l'autre.

L'une des faces **6** qui peut être, par exemple, une lame de verre, un film d'or fin ou un matériau polymère est fonctionnalisée par des sondes **8** spécifiques de cibles et l'autre face **7** est fonctionnalisée à l'aide de ligands **9** spécifiques de sécrétions cellulaires **10.**

Cette dernière face est compatible avec l'imagerie SPR afin de suivre la capture des molécules sécrétées par les cellules capturées sur la face opposée et peut être un support en verre recouvert d'un film d'or par exemple.

Dans le montage décrit à la Figure 3, la face dédiée à la capture cellulaire est couplée à une détection optique par microscopie conventionnelle sur la face inférieure tandis que les biomolécules sécrétées diffusantes dans le milieu sont capturées sur la face supérieure fonctionnalisée par des ligands spécifiques et détectée par SPR. Dans cette configuration, chaque face d'observation produit simultanément des informations différentes, traitant soit de la capture spécifique des cellules soit de la capture spécifique des sécrétions.

### 2.2. Dispositif avec tapis de nano-microsphères.

Dans ce dispositif, le support solide dont la surface est fonctionnalisée par des ligands se liant spécifiquement à des molécules sécrétées est un prisme Horiba - verre SF11 (n=1.71, haute qualité optique), dont la surface plane a été revêtue de 47 nm d'or sur 3 nm de titane pour l'adhésion sur verre **[18, 19].**

De plus, afin de permettre l'adhésion des billes de silice sur l'or, une monocouche auto-assemblée de mercaptopropyltriéthoxysilane ou mercaptopropyltriméthoxysilane (MPTS) est formée sur la surface dorée. Ce traitement a été réalisé en utilisant un bain à 10 mM de MPTS dans le toluène pendant 12 h, suivi par des rinçages abondants avec toluène, éthanol et eau puis par un séchage à température ambiante sous flux de gaz inerte.

Les particules de silice sphériques peuvent être constituées d'un mélange composé à 25% en nombre de particules dont le diamètre moyen est de 500 nm et 75% en nombre de particules dont le diamètre moyen est de 1 µm. En effet, une monocouche présentant une telle proportion en particules de 500 nm et de 1 µm est plus homogène en imagerie SPR qu'une monocouche purement composée de billes de 1 µm de diamètre.

Certaines des billes sont fonctionnalisées avec un type d'anticorps selon des protocoles bien connus de l'homme du métier tels que, par exemple, celui décrit par Moon *et al,* 1996 **[20].** Il est aussi possible d'assembler des billes présentant des fonctionnalisations et/ou nature différentes et de contrôler leur position sur la surface. Ainsi, il est possible, dès le dépôt d'obtenir des surfaces ayant des motifs sensibilisés à détecter des substances et cellules différentes (comme schématisé à la Figure 4).

Sur la Figure 4, les billes **11** ne sont pas fonctionnalisées et les billes **12** et **13** sont fonctionnalisées de façon à reconnaître spécifiquement des lymphocytes B (anticorps anti-CD19, la protéine CD19, ou autrement appelé eB4, étant une protéine exprimée à la membrane cellulaire et considérée comme un marqueur des lymphocytes B) et des lymphocytes T (anticorps anti-CD90, la protéine CD90, ou autrement appelée Thy étant une protéine exprimée à la membrane cellulaire et considérée comme un marqueur des lymphocytes T) respectivement. A noter que cette détection spécifique apportée par la fonctionnalisation des billes **12** et **13** est à croiser avec la reconnaissance spécifique de substances par la fonctionnalisation de l'or du support **14.**

A cet effet, un film compact de billes est réalisé sur la surface d'or du support solide préalablement fonctionnalisé par une monocouche auto-assemblée (SAM) de mercaptopropyltriéthoxysilane ou mercaptopropyltriméthoxysilane (MPTS) et ce, en utilisant le procédé décrit dans la demande internationale WO 2014/037559 **[17].** Dans ce procédé, on distingue 3 parties :
(i) un système permettant la dispense des particules en solution. Pour que le procédé fonctionne, les particules doivent impérativement flotter à la surface d'un liquide porteur tel que de l'eau
(ii) un convoyeur liquide pour transporter et agencer les particules afin de former un film compact. Ce convoyeur liquide s'écoule sur un plan incliné puis dans une zone horizontale appelée zone de transfert. La liaison entre le liquide porteur et le substrat est assurée par un pont capillaire.
(iii) le support solide tel que précédemment préparé et fonctionnalisé par une monocouche auto-assemblée (SAM) de mercaptopropyltriéthoxysilane ou mercaptopropyltriméthoxysilane (MPTS) comme précédemment décrit, sur lequel le film compact doit être transféré et mis en mouvement par un convoyeur.

Le procédé consiste donc à dispenser les particules à la surface du liquide porteur. Le liquide porteur véhicule les particules jusqu'à la zone de transfert. Les particules s'accumulent dans la zone de transfert puis remontent le plan incliné. Les particules présentes sur le plan incliné exercent alors une pression qui aide à l'ordonnancement des particules présentes dans la zone de transfert.

### RÉFÉRENCES

**[1]** Demande internationale WO 98/23960 au nom de Isis Innovation Ltd publiée le 4 juin 1998.
**[2]** Czerkinski et al, 1983, « A solid-phase enzyme-linked immunospot (ELISPOT) assay for enumeration of specific antibody-secreting cells », J. Immunol. Methods, vol. 65, pages 109-121.
**[3]** Prussin & Metcalfe, 1995, « Détection of intracytoplasmic cytokine using flow cytometry and directly conjugated anti-cytokine antibodies », J. Immunol. Methods, vol. 188, pages 117-128.
**[4]** Turcanu & Williams, 2001, « Cell identification and isolation on the basis of cytokine secretion: A novel tool for investigating immune responses », Nat. Med., vol. 7, pages 373-376.
**[5]** Manz et al, 1995, « Analysis and sorting of live cells according to secreted molecules, relocated to a cell-surface affinity matrix », Proc. Natl. Acad. Sci. USA, vol. 92, pages 1921-1925.
**[6]** Westerink & Ewing, 2008, « The PC12 cell as a Model for Neurosecretion », Acta Physiol., vol. 192, pages 273-285.
**[7]** Huang et al, 2011, « Micro- and Nanotechnologies for Study of Cell Secretion », Anal. Chem., vol. 83, pages 4393-4406.
**[8]** Demande internationale WO 2011/056643 au nom de The University of Michigan publiée le 12 mai 2011.
**[9]** Wu et al, 2013, « Optofluidic Platform for Real-Time Monitoring of Live Cell Secretory Activities Using Fano Resonance in Gold Nanoslits », Small, vol. 9, pages 3532-3540.
**[10]** Demande internationale WO 2006/060646 au nom de The University of Texas System publiée le 8 juin 2006.
**[11]** Guedon et al, 2000, « Characterization and Optimization of a Real-Time, Parallel, Label-Free, Polypyrrole-Based DNA Sensor by Surface Plasmon Resonance Imaging », Anal. Chem., vol. 72, pages 6003-6009.
**[12]** Demande de brevet US 2013/137085 au nom du CEA publiée le 30 mai 2013.
**[13]** Zhu et al, 2008, « A microdevice for multiplexed detection of T-cell-secreted cytokines », Lab Chip, vol. 8, pages 2197-2205.
**[14]** Lange et al, 2008, « Conducting polymers in chemical sensors and arrays », Anal. Chim. Acta, vol. 614, pages 1-26.
**[15]** Bardosova et al, 2010, « The Langmuir-Blodgett Approach to Making Colloidal Photonic Crystals from Silica Spheres », Adv. Mater., vol. 22, pages 3104-3124.
**[16]** Bauert et al, 2005, « Self-Assembling of Particle Monolayers by Spin-Coating », European Cells and Materials, vol. 10, suppl. 5, page BS2.
**[17]** Demande internationale WO 2014/037559 au nom du CEA publiée le 13 mars 2014.
**[18]** Grosjean et al, 2005, « A polypyrrole protein microarray for antibody-antigen interaction studies using a label-free detection process », Anal Biochem, vol. 347, pages 193-200.
**[19]** Suraniti et al, 2007, « Real-time detection of lymphocytes binding on an antibody chip using SPR imaging », Lab Chip, vol. 7, pages 1206-1208.
**[20]** Moon et al, 1996, « Formation of uniform aminosilane thin layers: an imine formation to measure relative surface density of the amine group », Langmuir, vol. 12, pages 4621-4624.

## Revendications

1. Procédé pour mesurer en temps réel la sécrétion d'un composé Cₒ par une cible Cᵢ, ledit procédé comprenant :
- la mise en culture en milieu liquide, dans une chambre de culture, d'une pluralité de cibles parmi lesquelles se trouve au moins une cible Cᵢ, ladite chambre de culture présentant :
i) au moins une 1^{ère} surface sur laquelle ladite cible Cᵢ est présente, la présence de ladite cible Cᵢ sur ladite 1^{ère} surface générant un 1^{er} signal, et
ii) au moins une 2^{nde} surface, différente de ladite 1^{ère} surface et non coplanaire avec ladite 1^{ère} surface, fonctionnalisée par au moins un ligand se liant spécifiquement audit composé Cₒ sécrété par ladite cible Cᵢ, la liaison spécifique entre ledit ligand et ledit composé Cₒ générant un 2^{nd} signal distinct dudit 1^{er} signal, et
- la détection en temps réel dudit 2^{nd} signal et éventuellement la détection dudit 1^{er} signal,
une cible étant un élément sécréteur comprenant une ou plusieurs cellules identiques ou différentes, de type eucaryote ou procaryote.

2. Procédé pour mesurer en temps réel la sécrétion d'un composé Cₒ par une cible Cᵢ, ledit procédé comprenant :
- la mise en culture en milieu liquide dans une chambre de culture d'une pluralité de cibles parmi lesquelles se trouve au moins une cible Cᵢ, ladite chambre de culture présentant :
i) au moins une 1^{ère} surface fonctionnalisée par au moins une sonde se liant spécifiquement à ladite cible Cᵢ, la liaison spécifique entre ladite sonde et ladite cible Cᵢ générant un 1^{er} signal, et
ii) au moins une 2^{nde} surface, différente de ladite 1^{ère} surface et non coplanaire avec ladite 1^{ère} surface, fonctionnalisée par au moins un ligand se liant spécifiquement audit composé Cₒ sécrété par ladite cible Cᵢ liée à ladite sonde, la liaison spécifique entre ledit ligand et ledit composé Cₒ générant un 2^{nd} signal distinct dudit 1^{er} signal, et
- la détection en temps réel dudit 2^{nd} signal et éventuellement la détection dudit 1^{er} signal,
une cible étant un élément sécréteur comprenant une ou plusieurs cellules identiques ou différentes, de type eucaryote ou procaryote.

3. Procédé selon la revendication 1 ou 2, **caractérisé en ce que** ladite cible et ledit ligand sont distants l'un de l'autre de 100 nm à 500 µm et avantageusement d'au moins 300 nm.

4. Procédé selon l'une quelconque des revendications 1 à 4 3, **caractérisé en ce que** ladite cible Cᵢ est (i) une cellule individuelle, (ii) un ensemble de cellules identiques tel qu'un groupement de cellules identiques encapsulées, (iii) un ensemble de cellules d'au moins deux types différents tel qu'un tissu ou fragment de tissu ou encore (iv) un ensemble de cellules d'au moins deux types différents encapsulées.

5. Procédé selon l'une quelconque des revendications 2 à 4, **caractérisé en ce que** ladite sonde est choisie dans le groupe constitué par un peptide ; un oligopeptide ; une protéine; une glycoprotéine ; un oligosaccharide; un polysaccharide; un glucide; une lipoprotéine; un lipide; un phospholipide; un agoniste ou un antagoniste d'un récepteur membranaire ; un anticorps polyclonal ou monoclonal ; un fragment d'anticorps tel qu'un fragment Fab, F(ab')₂, Fv, scFv, diabody ou un domaine hypervariable (ou CDR pour « Complementarity Determining Région ») ; une molécule nucléotidique ; un acide nucléique peptidique ; un aptamère tel qu'un aptamère ADN ou un aptamère ARN et un polymère adapté à la fixation de ces cibles tel qu'un polymère du type poly(N-isopropylacrylamide) (pNIPAM), un polymère du type pNIPAM-co-acrylamidophénylboronate (pNIPAM-co-APBA), un polypyrrole, une polylysine, un hydrocarbure aromatique polycyclique (PAH), un polyétherimide (PEI) ou un acide polyacrylique (PAA).

6. Procédé selon l'une quelconque des revendications 1 à 5, **caractérisé en ce que** ledit composé Cₒ est ou comprend un élément choisi dans le groupe constitué par une protéine, un polypeptide, un peptide, un lipide, une glycoprotéine, un glycolipide, une lipoprotéine, un ion inorganique, une petite molécule organique comprenant de 1 à 100 atomes de carbone et un composé particulaire ou supramoléculaire, tel qu'une vésicule, un exosome, un organisme microbien comme, par exemple, un virus ou une particule microbienne comme, par exemple, une particule virale.

7. Procédé selon l'une quelconque des revendications 1 à 6, **caractérisé en ce que** ledit ligand est choisi dans le groupe constitué par un peptide ; un oligopeptide ; une protéine ; une glycoprotéine ; un oligosaccharide ; un polysaccharide; un glucide; une lipoprotéine; un lipide; un phospholipide ; un anticorps polyclonal ou monoclonal ; un fragment d'anticorps tel qu'un fragment Fab, F(ab')₂, Fv, scFv, diabody ou un domaine hypervariable (ou CDR pour « Complementarity Determining Région ») ; un haptène ; une molécule nucléotidique telle que précédemment définie ; un acide nucléique peptidique ; un aptamère tel qu'un aptamère ADN ou un aptamère ARN, un polymère adapté à la fixation spécifique d'ions inorganiques et un polymère adapté à la fixation de ces cibles tel qu'un polymère du type poly(N-isopropylacrylamide) (pNIPAM), un polymère du type pNIPAM-co-acrylamidophénylboronate (pNIPAM-co-APBA), un polypyrrole, une polylysine, un hydrocarbure aromatique polycyclique (PAH), un polyétherimide (PEI) ou un acide polyacrylique (PAA).

8. Procédé selon l'une quelconque des revendications 1 à 7, **caractérisé en ce que** ladite 1^{ère} et ladite 2^{nde} surfaces appartiennent à deux supports solides différents présents dans ladite chambre de culture.

9. Procédé selon l'une quelconque des revendications 1 à 7, **caractérisé en ce que** ladite 1^{ère} et ladite 2^{nde} surfaces dépendent d'un même support solide présentant une structuration physique impliquant des éléments en relief.

10. Procédé selon l'une quelconque des revendications 1 à 7, **caractérisé en ce que** plusieurs particules solides sont disposées sur un support solide présentant au moins une 2^{nde} surface, tout ou partie de la surface d'au moins une desdites particules solides correspondant à une 1^{ère} surface et lesdites particules solides formant une couche poreuse.

11. Procédé selon l'une quelconque des revendications 1 à 10, **caractérisé en ce que** lesdits supports solides, lesdits des éléments en relief, lesdites particules, ladite 1^{ère} surface et/ou ladite 2^{nde} surface sont des supports solides, des éléments en relief, des particules et/ou des surfaces en matériau inorganique notamment choisi dans le groupe constitué par les verres, les quartz, les céramiques, les métaux, les métalloïdes, les carbones allotropiques et leurs mélanges.

12. Procédé selon l'une quelconque des revendications 1 à 10, **caractérisé en ce que** lesdits supports solides, lesdits des éléments en relief, lesdites particules, ladite 1^{ère} surface et/ou ladite 2^{nde} surface sont des supports solides, des éléments en relief, des particules et/ou des surfaces en matériau organique, notamment choisi dans le groupe constitué par de l'agarose, un polyamide (type nylon), un polycarbonate, un polyéthylène glycol, un fluoropolymère, un acrylate, un siloxane (polydiméthylsiloxane; PDMS), un copolymère oléfinique cyclique (COC), un polyéther éther cétone (PEEK) et de la nitro-cellulose.

13. Procédé selon l'une quelconque des revendications 1 à 10, **caractérisé en ce qu'**au moins une surface choisie entre ladite 1^{ère} surface et ladite 2^{nde} surface est en un matériau organique, notamment choisi dans le groupe constitué par de l'agarose, un polyamide (type nylon), un polycarbonate, un polyéthylène glycol, un fluoropolymère, un acrylate, un siloxane (polydiméthylsiloxane ; PDMS), un copolymère oléfinique cyclique (COC), un polyéther éther cétone (PEEK) et de la nitro-cellulose, l'autre surface étant en un matériau inorganique notamment choisi dans le groupe constitué par les verres, les quartz, les céramiques, les métaux, les métalloïdes, les carbones allotropiques et leurs mélanges.

14. Procédé selon l'une quelconque des revendications 1 à 13, **caractérisé en ce que** la technique utilisée pour détecter le 2^{nd} signal est choisie dans le groupe constitué par la résonance des plasmons de surface en mode monopoint, la résonance des plasmons de surface en imagerie, une technique optique en champ proche et la mesure d'impédance.

15. Procédé selon l'une quelconque des revendications 1 à 14, **caractérisé en ce que** la technique utilisée pour détecter le 1^{er} signal est choisie dans le groupe constitué par une technique optique telle que, par exemple, la microscopie optique, la microscopie optique en mode guidé ou la microscopie de fluorescence.

16. Dispositif susceptible d'être mis en oeuvre dans un procédé tel que défini à l'une quelconque des revendications 1 à 8 et 11 à 15, ledit dispositif comprenant une chambre de culture dans laquelle
une 1^{ère} surface éventuellement fonctionnalisée par au moins une sonde se liant spécifiquement à une cible Cᵢ et
une 2^{nde} surface, différente de ladite 1^{ère} surface et non coplanaire avec ladite 1^{ère} surface, fonctionnalisée par au moins un ligand se liant spécifiquement à un composé Cₒ sécrété par ladite cible Cᵢ éventuellement liée à ladite sonde
appartiennent à deux supports solides différents.

17. Dispositif susceptible d'être mis en oeuvre dans un procédé tel que défini à l'une quelconque des revendications 1 à 8 7 et 10 à 15, ledit dispositif comprenant une chambre de culture dans laquelle plusieurs particules solides sont disposées sur un support solide et forment une couche poreuse,
tout ou partie de la surface d'au moins une des particules solides correspondant à une 1^{ère} surface éventuellement fonctionnalisée par au moins une sonde se liant spécifiquement à une cible Cᵢ et
ledit support solide présentant au moins une 2^{nde} surface, différente de ladite 1^{ère} surface et non coplanaire avec ladite 1^{ère} surface, fonctionnalisée par au moins un ligand se liant spécifiquement à un composé Cₒ sécrété par ladite cible Cᵢ éventuellement liée à ladite sonde.

18. Dispositif selon la revendication 17, **caractérisé en ce que** ledit support solide est fonctionnalisé chimiquement pour permettre l'assemblage covalent ou non covalent desdites particules solides.

19. Dispositif selon la revendication 17 ou 18, **caractérisé en ce qu'**un bras espaceur du type monocouche auto-assemblée (SAM) de mercaptopropyltriéthoxysilane ou mercaptopropyltriméthoxysilane (MPTS) a été préalablement immobilisé sur le support solide pour permettre l'accrochage desdites particules solides.

## Patentansprüche

1. Verfahren zur Echtzeitmessung der Sekretion einer Verbindung Cₒ durch ein Sekretionsziel Cᵢ, wobei das Verfahren umfasst:
- Kultivieren einer Vielzahl von Zielen, unter denen sich zumindest ein Ziel Cᵢ befindet, in einem flüssigen Medium in einer Kultivierkammer, wobei die Kultivierkammer aufweist:
i) zumindest eine erste Fläche, auf der das Ziel Cᵢ vorhanden ist, wobei das Vorhandensein des Ziels Cᵢ auf der ersten Fläche ein erstes Signal erzeugt, und
ii) zumindest eine zweite Fläche, die sich von der ersten Fläche unterscheidet und nicht koplanar mit der ersten Fläche verläuft und funktionalisiert ist durch zumindest einen Liganden, der sich spezifisch an die durch das Ziel Cᵢ sekretierte Verbindung Cₒ bindet, wobei die spezifische Bindung zwischen dem Liganden und der Verbindung Cₒ ein zweites Signal erzeugt, das sich von dem ersten Signal unterscheidet, und
- Erfassen des zweiten Signals in Echtzeit und gegebenenfalls Erfassen des ersten Signals,
wobei ein Ziel ein sekretorisches Element ist, das eine oder mehrere identische oder verschiedene Zellen vom eukaryotischen oder prokaryontischen Typ enthält.

2. Verfahren zur Echtzeitmessung der Sekretion einer Verbindung Cₒ durch ein Sekretionsziel Cᵢ, wobei das Verfahren umfasst:
- Kultivieren einer Vielzahl von Zielen, unter denen sich zumindest ein Ziel Cᵢ befindet, in einem flüssigen Medium in einer Kultivierkammer, wobei die Kultivierkammer aufweist:
i) zumindest eine erste Fläche, die durch zumindest eine Sonde funktionalisiert ist, die sich spezifisch an das Ziel Cᵢ bindet, wobei die spezifische Bindung zwischen der Sonde und dem Ziel Cᵢ ein erstes Signal erzeugt, und
ii) zumindest eine zweite Fläche, die sich von der ersten Fläche unterscheidet und nicht koplanar mit der ersten Fläche verläuft und funktionalisiert ist durch zumindest einen Liganden, der sich spezifisch an die Verbindung Cₒ bindet, die durch das Ziel Cᵢ, sekretiert ist, das an die Sonde gebunden ist, wobei die spezifische Bindung zwischen dem Liganden und der Verbindung Cₒ ein zweites Signal erzeugt, das sich von dem ersten Signal unterscheidet, und
- Erfassen des zweiten Signals in Echtzeit und gegebenenfalls Erfassen des ersten Signals,
wobei ein Ziel ein sekretorisches Element ist, das eine oder mehrere identische oder verschiedene Zellen vom eukaryotischen oder prokaryontischen Typ enthält.

3. Verfahren nach Anspruch 1 oder 2, **dadurch gekennzeichnet, dass** das Ziel und der Ligand um 100 nm bis 500 µm und vorteilhaft um mindestens 300 nm voneinander beabstandet sind.

4. Verfahren nach einem der Ansprüche 1 bis 3, **dadurch gekennzeichnet, dass** das Ziel Cᵢ (i) eine einzelne Zelle, (ii) eine Anordnung von identischen Zellen, wie etwa eine Gruppe von eingekapselten, identischen Zellen, (iii) eine Anordnung von Zellen von zumindest zwei verschiedenen Arten, wie etwa ein Gewebe oder Gewebefragment, oder auch (iv) eine Anordnung von Zellen von zumindest zwei verschiedenen, eingekapselten Arten ist.

5. Verfahren nach einem der Ansprüche 2 bis 4, **dadurch gekennzeichnet, dass** die Sonde ausgewählt ist aus der Gruppe umfassend ein Peptid; ein Oligopeptid; ein Protein; ein Glycoprotein; ein Oligosaccarid; ein Polysaccharid; ein Glucid; ein Lipoprotein; ein Lipid; ein Phospholipid; einen Agonist oder Antagonist eines Membranrezeptors; einen polyklonalen oder monoklonalen Antikörper; ein Antikörperfragment, wie ein Fragment Fab, F(ab')₂, Fv, scFv, ein Diabody oder eine hypervariable Domäne (oder CDR für "Complementarity Determining Region"); ein Nukleotidmolekül; eine Peptidnukleinsäure; ein Aptamer, wie etwa ein DNA-Aptamer oder ein RNA-Aptamer und ein zur Fixierung dieser Ziele geeignetes Polymer, wie etwa ein Polymer des Typs Poly(N-isopropylacrylamid) (pNIPAM), ein Polymer des Typs pNIPAM-co-acrylamidophenylboronat (pNIPAM-co-APBA), ein Polypyrrol, ein Polylysin, einen polycyclischen aromatischen Kohlenwasserstoff (PAH), ein Polyetherimid (PEI) oder eine Polyacrylsäure (PAA).

6. Verfahren nach einem der Ansprüche 1 bis 5, **dadurch gekennzeichnet, dass** die Verbindung Cₒ ein Element ist oder enthält, ausgewählt aus der Gruppe umfassend ein Protein, ein Polypeptid, ein Peptid, ein Lipid, ein Glycoprotein, ein Glycolipid, ein Lipoprotein, ein organisches Ion, ein kleines organisches Molekül mit 1 bis 100 Kohlenstoffatomen und eine teilchenförmige oder supramolekulare Verbindung, wie etwa ein Vesikel, ein Exosom, ein mikrobieller Organismus, wie beispielsweise ein Virus oder ein mikrobieller Partikel, wie beispielsweise ein Viruspartikel.

7. Verfahren nach einem der Ansprüche 1 bis 6, **dadurch gekennzeichnet, dass** der Ligand ausgewählt ist aus der Gruppe umfassend ein Peptid; ein Oligopeptid; ein Protein; ein Glycoprotein; ein Oligosaccarid; ein Polysaccharid; ein Glucid; ein Lipoprotein; ein Lipid; ein Phospholipid; einen polyklonalen oder monoklonalen Antikörper; ein Antikörperfragment, wie ein Fragment Fab, F(ab')₂, Fv, scFv, ein Diabody oder eine hypervariable Domäne (oder CDR für "Complementarity Determining Region"); ein Hapten; ein Nukleotidmolekül wie vorangehend definiert; eine Peptidnukleinsäure; ein Aptamer, wie etwa ein DNA-Aptamer oder ein RNA-Aptamer, und ein zur spezifischen Fixierung von anorganischen Ionen geeignetes Polymer und ein zur Fixierung dieser Ziele geeignetes Polymer, wie etwa ein Polymer des Typs Poly(N-isopropylacrylamid) (pNIPAM), ein Polymer des Typs pNIPAM-co-acrylamidophenylboronat (pNIPAM-co-APBA), ein Polypyrrol, ein Polylysin, einen polycyclischen aromatischen Kohlenwasserstoff (PAH), ein Polyetherimid (PEI) oder eine Polyacrylsäure (PAA).

8. Verfahren nach einem der Ansprüche 1 bis 7, **dadurch gekennzeichnet, dass** die erste und die zweite Fläche zwei verschiedenen, festen Trägern angehören, die in der Kultivierkammer vorhanden sind.

9. Verfahren nach einem der Ansprüche 1 bis 7, **dadurch gekennzeichnet, dass** die erste und die zweite Fläche von einem selben festen Träger abhängen, der eine physische Strukturierung aufweist, die hervorgehobene Elemente umfasst.

10. Verfahren nach einem der Ansprüche 1 bis 7, **dadurch gekennzeichnet, dass** mehrere feste Teilchen auf einem festen Träger angeordnet sind, der zumindest eine zweite Fläche aufweist, wobei die gesamte Fläche oder eine Teilfläche zumindest eines der festen Teilchen einer ersten Fläche entspricht und die festen Teilchen eine poröse Schicht bilden.

11. Verfahren nach einem der Ansprüche 1 bis 10, **dadurch gekennzeichnet, dass** die festen Träger, die hervorgehobenen Elemente, die Teilchen, die erste Fläche und/oder die zweite Fläche feste Träger, hervorgehobene Elemente, Teilchen und/oder Flächen aus anorganischem Material sind, insbesondere ausgewählt aus der Gruppe umfassend Gläser, Quarze, Keramiken, Metalle, Metalloide, allotrope Kohlenstoffe und deren Gemische.

12. Verfahren nach einem der Ansprüche 1 bis 10, **dadurch gekennzeichnet, dass** die festen Träger, die hervorgehobenen Elemente, die Teilchen, die erste Fläche und/oder die zweite Fläche feste Träger, hervorgehobene Elemente, Teilchen und/oder Flächen aus organischem Material sind, insbesondere ausgewählt aus der Gruppe umfassend Agarose, ein Polyamid (Typ Nylon), ein Polycarbonat, ein Polyethylenglycol, ein Fluorpolymer, ein Acrylat, ein Siloxan (Polydimethylsiloxan; PDMS), ein cyclisches Olefincopolymer (COC), ein Polyetheretherketon (PEEK) und Nitrozellulose.

13. Verfahren nach einem der Ansprüche 1 bis 10, **dadurch gekennzeichnet, dass** zumindest eine Fläche, ausgewählt aus der ersten Fläche und der zweiten Fläche, aus einem organischen Material besteht, insbesondere ausgewählt aus der Gruppe umfassend Agarose, ein Polyamid (Typ Nylon), ein Polycarbonat, ein Polyethylenglycol, ein Fluorpolymer, ein Acrylat, ein Siloxan (Polydimethylsiloxan; PDMS), ein cyclisches Olefincopolymer (COC), ein Polyetheretherketon (PEEK) und Nitrozellulose, wobei die weitere Fläche aus einem anorganischen Material besteht, insbesondere ausgewählt aus der Gruppe umfassend Gläser, Quarze, Keramiken, Metalle, Metalloide, allotrope Kohlenstoffe und deren Gemische.

14. Verfahren nach einem der Ansprüche 1 bis 13, **dadurch gekennzeichnet, dass** die verwendete Technik zum Erfassen des zweiten Signals ausgewählt aus der Gruppe umfassend die Oberflächenplasmonresonanz im Einpunkt-Modus, die Oberflächenplasmonresonanz zur Bildgebung, eine optische Nahfeldtechnik und die Impedanzmessung.

15. Verfahren nach einem der Ansprüche 1 bis 14, **dadurch gekennzeichnet, dass** die verwendete Technik zum Erfassen des ersten Signals ausgewählt ist aus der Gruppe umfassend eine optische Technik, wie beispielsweise die optische Mikroskopie, die optische Mikroskopie im geführten Modus oder die Fluoreszenzmikroskopie.

16. Vorrichtung zum Einsetzen bei einem Verfahren nach einem der Ansprüche 1 bis 8 und 11 bis 15, wobei die Vorrichtung eine Kultivierkammer enthält, in welcher
eine erste Fläche, gegebenenfalls funktionalisiert durch zumindest eine Sonde, die sich spezifisch an ein Ziel Cᵢ bindet, und
eine zweite Fläche, die sich von der ersten Fläche unterscheidet und nicht koplanar mit der ersten Fläche verläuft und funktionalisiert ist durch zumindest einen Liganden, der sich spezifisch an die Verbindung Cₒ bindet, die durch das Ziel Cᵢ sekretiert ist, das gegebenenfalls an die Sonde gebunden ist,
zwei verschiedenen festen Trägern angehören.

17. Vorrichtung zum Einsetzen bei einem Verfahren nach einem der Ansprüche 1 bis 7 und 10 bis 15, wobei die Vorrichtung eine Kultivierkammer enthält, in welcher mehrere feste Teilchen an einem festen Träger angeordnet sind und eine poröse Schicht bilden,
wobei
wobei die gesamte Fläche oder eine Teilfläche zumindest eines der festen Teilchen einer ersten Fläche entspricht, die gegebenenfalls funktionalisiert ist durch zumindest eine Sonde, die sich spezifisch an ein Ziel Cᵢ bindet, und
der feste Träger zumindest eine zweite Fläche aufweist, die sich von der ersten Fläche unterscheidet und nicht koplanar mit der ersten Fläche verläuft und funktionalisiert ist durch zumindest einen Liganden, der sich spezifisch an eine Verbindung Cₒ bindet, die durch das Ziel Cᵢ sekretiert ist, das gegebenenfalls an die Sonde gebunden ist.

18. Vorrichtung nach Anspruch 17, **dadurch gekennzeichnet, dass** der feste Träger chemisch funktionalisiert ist, um das kovalente oder nicht kovalente Zusammenfügen der festen Teilchen zu gestatten.

19. Vorrichtung nach Anspruch 17 oder 18, **dadurch gekennzeichnet, dass** ein Abstandshalterarm vom Typ selbstorganisierter Monoschicht (SAM) aus Mercapropyltriethoxysilan oder Mecraptopropyltrimethoxysilan (MPTS) zuvor auf dem festen Träger immobilisiert wurde, um die Anlagerung fester Teilchen zu ermöglichen.

## Claims

1. Method for measuring in real time the secretion of a compound Cₒ by a target Cᵢ said method comprising:
- the culture in a liquid medium, in a culture chamber, of a plurality of targets among which is found at least one target Cᵢ said culture chamber having:
i) at least one 1^{st} surface on which said target Cᵢ is present, the presence of said target Cᵢ on said 1^{st} surface generating a 1^{st} signal, and
ii) at least one 2^{nd} surface, different from said 1^{st} surface and non-coplanar with said 1^{st} surface, functionalized with at least one ligand specifically binding to said compound Cₒ secreted by said target Q, the specific binding between said ligand and said compound Cₒ generating a 2^{nd} signal distinct from said 1^{st} signal, and
- the real time detection of said 2^{nd} signal and optionally the detection of said 1^{st} signal,
a target being a secreting element comprising one or several, eukaryotic or prokaryotic type, identical or different cells.

2. A method for measuring in real time the secretion of a compound Cₒ by a target Cᵢ, said method comprising:
- the culture in a liquid medium in a culture chamber of a plurality of targets among which is found at least one target Q, said culture chamber having:
i) at least one 1^{st} surface functionalized with at least one probe specifically binding to said target Cᵢ, the specific binding between said probe and said target Cᵢ generating a 1^{st} signal, and
ii) at least one 2^{nd} surface, different from said 1^{st} surface and non-coplanar with said 1^{st} surface, functionalized by at least one ligand specifically binding to said compound Cₒ secreted by said target Cᵢ bound to said probe, the specific binding between said ligand and said compound Cₒ generating a 2^{nd} signal distinct from said 1^{st} signal, and
- the real time detection of said 2^{nd} signal and optionally the detection of said 1^{st} signal,
a target being a secreting element comprising one or several, eukaryotic or prokaryotic type, identical or different cells.

3. The method according to claim 1 or 2, **characterized in that** said target and said ligand are distant from one another by 100 nm to 500 µm and advantageously by at least 300 nm.

4. The method according to any of claims 1 to 3, **characterized in that** said target Cᵢ is (i) an individual cell, (ii) a set of identical cells such as a group of encapsulated identical cells, (iii) a set of cells of at least two different types such as a tissue or a tissue fragment or further (iv) a set of cells of at least two different encapsulated types.

5. The method according to any of claims 2 to 4, **characterized in that** said probe is selected from the group consisting of a peptide; an oligopeptide; a protein; a glycoprotein; an oligosaccharide; a polysaccharide; a carbohydrate; a lipoprotein; a lipid; a phospholipid; an agonist or antagonist of a membrane receptor; a polyclonal or monoclonal antibody; an antibody fragment such as a fragment Fab, F(ab')₂, Fv, scFv, diabody or a hypervariable domain (or CDR for "Complementarity Determining Region") ; a nucleotide molecule; a peptide nucleic acid; an aptamer such as a DNA aptamer or an RNA aptamer and a polymer adapted to the attachment of these targets such as a polymer of the poly(N-isopropylacrylamide) (pNIPAM) type, a polymer of the pNIPAM-co-acrylamidophenylboronate (pNIPAM-co-APBA) type, a polypyrrol, a polylysine, a polycyclic aromatic hydrocarbon (PAH), a polyetherimide (PEI) or a polyacrylic acid (PAA).

6. The method according to any of claims 1 to 5, **characterized in that** said compound Cₒ is or comprises an element selected from the group consisting of a protein, a polypeptide, a peptide, a lipid, a glycoprotein, a glycolipid, a lipoprotein, an inorganic ion, a small organic molecule comprising from 1 to 100 carbon atoms and a particulate or supramolecular compound, such as a vesicle, an exosome, a microbial organism such as for example a virus or a microbial particle like for example a viral particle.

7. The method according to any of claims 1 to 6, **characterized in that** said ligand is selected from the group consisting of a peptide; an oligopeptide; a protein; a glycoprotein; an oligosaccharide; a polysaccharide; a carbohydrate; a lipoprotein; a lipid; a phospholipid; a polyclonal or monoclonal antibody; an antibody fragment such as a fragment Fab, F(ab')₂, Fv, scFv, diabody or a hypervariable domain (or CDR for "Complementarity Determining Region"); a haptene; a nucleotide molecule as previously defined; a peptide nucleic acid; an aptamer such as a DNA aptamer or an RNA aptamer, a polymer adapted to the specific attachment of inorganic ions and a polymer adapted to the attachment of these targets such as a polymer of the poly(N-isopropylacrylamide) (pNIPAM) type, a polymer of the pNIPAM-co-acrylamidophenylboronate (pNIPAM-co-APBA) type, a polypyrrol, a polylysine, a polycyclic aromatic hydrocarbon (PAH), a polyetherimide (PEI) or a polyacrylic acid (PAA).

8. The method according to any of claims 1 to 7, **characterized in that** said 1^{st} and said 2^{nd} surfaces belong to two different solid supports present in said culture chamber.

9. The method according to any of claims 1 to 7, **characterized in that** said 1^{st} and said 2^{nd} surfaces depend on a same solid support having a physical structuration involving embossed elements.

10. The method according to any of claims 1 to 7, **characterized in that** several solid particles are positioned on a solid support having at least one 2^{nd} surface, all or part of the surface of at least one of said solid particles corresponding to a 1^{st} surface and said solid particles forming a porous layer.

11. The method according to any of claims 1 to 10, **characterized in that** said solid supports, said embossed elements, said particles, said 1^{st} surface and/or said 2^{nd} surface are solid supports, embossed elements, particles and/or surfaces in an inorganic material notably selected from the group consisting of glasses, quartzes, ceramics, metals, metalloids, allotropic carbons and mixtures thereof.

12. The method according to any of claims 1 to 10, **characterized in that** said solid supports, said embossed elements, said particles, said 1^{st} surface and/or said 2^{nd} surface are solid supports, embossed elements, particles and/or surfaces in an organic material, notably selected from the group consisting of agarose, polyamide (nylon type), polycarbonate, polyethylene glycol, fluoropolymer, acrylate, a siloxane (polydimethylsiloxane; PDMS), a cyclic olefin copolymer (COC), a polyether-ether-ketone (PEEK) and nitro-cellulose.

13. The method according to any of claims 1 to 10, **characterized in that** at least one surface selected from between said 1^{st} surface and said 2^{nd} surface is in an organic material, notably selected from the group consisting of agarose, a polyamide (nylon type), a polycarbonate, a polyethylene glycol, a fluoropolymer, an acrylate, a siloxane (polydimethylsiloxane; PDMS), a cyclic olefin copolymer (COC), a polyether-ether-ketone (PEEK) and nitro-cellulose, the other surface being in an inorganic material notably selected from the group consisting of glasses, quartzes, ceramics, metals, metalloids, allotropic carbons and mixtures thereof.

14. The method according to any of claims 1 to 13, **characterized in that** the technique used for detecting the 2^{nd} signal is selected from the group consisting of surface plasmon resonance in a single point mode, the surface plasmon resonance in imaging, an optical technique in a near field and the measurement of impedance.

15. The method according to any of claims 1 to 14, **characterized in that** the technique used for detecting the 1^{st} signal is selected from the group consisting of an optical technique such as for example optical microscopy, optical microscopy in a guided mode or fluorescence microscopy.

16. A device which may be implemented in a method as defined in any of claims 1 to 8 and 11 to 15, said device comprising a culture chamber wherein
a 1^{st} surface optionally functionalized with at least one probe specifically binding to a target Cᵢ and
a 2^{nd} surface, different from said 1^{st} surface and non-coplanar with said 1^{st} surface, functionalized by at least one ligand specifically binding to a compound Cₒ secreted by said target Cᵢ optionally bound to said probe
belong to two different solid supports.

17. A device which may be implemented in a method as defined in any of claims 1 to 7 and 10 to 15, said device comprising a culture chamber wherein several solid particles are positioned on a solid support and form a porous layer,
all or part of the surface of at least one of the solid particles corresponding to a 1^{st} surface optionally functionalized with at least one probe specifically binding to a target Cᵢ and
said solid support having at least one 2^{nd} surface, different from said 1^{st} surface and non-coplanar with said 1^{st} surface, functionalized by at least one ligand specifically binding to a compound Cₒ secreted by said target Cᵢ optionally bound to said probe.

18. The device according to claim 17, **characterized in that** said solid support is chemically functionalized for allowing the covalent or non-covalent assembling of said solid pa rticles.

19. The device according to claim 17 or 18, **characterized in that** a spacer arm of the self-assembled monolayer type (SAM) of mercaptopropyltriethoxysilane or mercaptopropyltrimethoxysilane (MPTS) was immobilized beforehand on the solid support for giving the possibility of hooking-up said solid particles.
